# EUROPÄISCHE PATENTSCHRIFT

(11) **EP 2 253 226 B1**
(45) Veröffentlichungstag und Bekanntmachung des Hinweises auf die Patenterteilung: **15.07.2015**
(21) Anmeldenummer: 10162980.6
(22) Anmeldetag: 17.05.2010
(51) Int. Cl.: A23L 1/226, A23L 1/236, A23L 1/22, C07D 311/60

(54) **Verwendung von Hydroxyflavan-Derivaten zur Geschmacksmodifizierung**
Use of hydroxyflavone derivatives for modifying taste
Utilisation de dérivés d'hydroxyflavane pour la modification du goût

(30) Priorität: 15.05.2009 US 178667 P
(43) Veröffentlichungstag der Anmeldung: 24.11.2010
(73) Patentinhaber: Leibniz-Institut für Pflanzenbiochemie (IPB), 06120 Halle (Saale) (DE); Symrise AG, 37603 Holzminden (DE)
(72) Erfinder: Wessjohann, Ludger, 06120, Halle (Saale) (DE); Backes, Michael, 37603, Holzminden (DE); Ley, Jakob, 37603, Holzminden (DE); Paetz, Susanne, 37671, Höxter (DE); Reichelt, Katharina, 83026, Rosenheim (DE)
(74) Vertreter: Fabry, Bernd

(56) Entgegenhaltungen:
- WO-A2-2009/149288
- US-A- 6 054 129
- KAMARA B I ET AL: "Phenolic metabolites from Honeybush Tea (Cyclopia subternata" JOURNAL OF AGRICULTURAL AND FOOD CHEMISTRY, AMERICAN CHEMICAL SOCIETY, US LNKD- DOI:10.1021/JF040097Z, Bd. 52, Nr. 17, 28. Juli 2004 (2004-07-28) , Seiten 5391-5395, XP002368627 ISSN: 0021-8561

## Beschreibung

Die vorliegende Erfindung betrifft primär die Verwendung von Verbindungen der Formel (I), deren Salzen oder Mischungen davon zur synergistischen Verstärkung des süßen Geschmacks eines süß schmeckenden Stoffes.

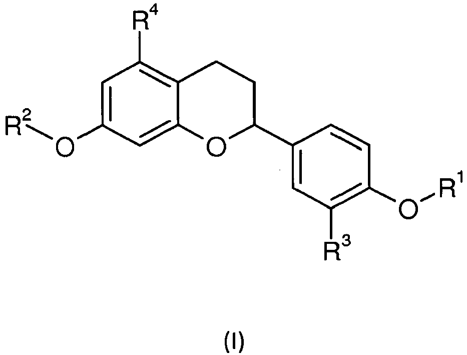

Die Bedeutung der Reste R¹ bis R⁴ und die Konfiguration am chiralen Kohlenstoffatom sowie bevorzugte Verbindungen der Formel (I) sind weiter unten beschreiben.

Ein weiterer Aspekt der vorliegenden Erfindung betrifft die Verwendung von Verbindungen der Formel (I), deren Salzen oder Mischungen davon zum Maskieren oder Vermindern unangenehmer Geschmackseindrücke, insbesondere zum Maskieren oder Vermindern eines bitteren Geschmackseindrucks eines bitter schmeckenden Stoffes.

Die vorliegende Erfindung betrifft zudem eine Aromakomposition umfassend eine oder mehrere Verbindungen der Formel (I), deren Salze oder Mischungen davon.

Ferner betrifft die vorliegende Erfindung Zubereitungen, insbesondere der Ernährung, der Mundpflege oder dem Genuss dienende oder kosmetische oder zur oralen Aufnahme bestimmte pharmazeutische Zubereitungen umfassend eine oder mehrere Verbindungen der Formel (I), deren Salze oder Mischungen davon.

Zudem betrifft die vorliegende Erfindung ein Verfahren zum synergistischen Verstärken des süßen Geschmacks eines süß schmeckenden Stoffes.

Weitere Aspekte der vorliegenden Erfindung und bevorzugte Ausgestaltungen davon ergeben sich aus der folgenden Beschreibung und den beigefügten Patentansprüchen.

Nahrungs- oder Genussmittel, die einen hohen Zuckergehalt (vor allem Sucrose (= Saccharose), Lactose, Glucose oder Fructose oder deren Mischungen) aufweisen, werden in der Regel von Verbrauchern auf Grund ihrer Süße stark präferiert. Auf der anderen Seite ist allgemein bekannt, dass ein hoher Gehalt an leicht verstoffwechselbaren Kohlenhydraten den Blutzuckerspiegel stark ansteigen lässt, zur Bildung von Fettdepots führt und letztendlich zu gesundheitlichen Problemen wie Übergewicht, Fettleibigkeit, Insulinresistenz, Altersdiabetes und deren Folgeerkrankungen führen kann. Insbesondere kommt erschwerend hinzu, dass viele der oben genannten Kohlenhydrate zusätzlich die Zahngesundheit beeinträchtigen können, da sie von bestimmten Bakteriensorten in der Mundhöhle zu beispielsweise Milchsäure abgebaut werden und den Zahnschmelz der juvenilen oder adulten Zähne angreifen können (Karies).

Daher ist es schon lange ein Ziel, den Zuckergehalt von Nahrungs- und/oder Genussmitteln auf das unbedingt nötige Maß oder darunter zu reduzieren. Eine entsprechende Maßnahme besteht im Einsatz von Süßstoffen: das sind chemisch einheitliche Substanzen, die selbst keinen oder nur einen sehr geringen kalorischen Brennwert haben und gleichzeitig einen starken süßen Geschmackseindruck verursachen; die Stoffe sind in der Regel nicht-kariogen (eine Übersicht ist z.B. zu finden in Journal of the American Dietetic Association 2004, 104 (2), 255-275).

Die sogenannten Bulk-Süßstoffe wie Sorbitol, Mannitol oder andere Zuckeralkohole sind zwar teilweise ausgezeichnete Süßungsmittel und können auch die übrigen nahrungsmitteltechnischen Eigenschaften von Zuckern teilweise ersetzen, führen aber bei zu häufiger Aufnahme bei einem Anteil der Bevölkerung zu osmotisch bedingten Verdauungsproblemen.

Nicht-nutritive, hochintensive Süßstoffe sind zwar durch ihre geringe Einsatzkonzentration gut geeignet, Süße in Nahrungsmittel zu bringen, zeigen jedoch oft geschmackliche Probleme durch im Vergleich zu Zucker unähnliche Zeit-Intensitätsprofile (z.B. Sucralose, Stevioside, Cyclamat), einen bitteren und/oder adstringierenden Nachgeschmack (z.B. Acesulfam K, Saccharin, Stevioside, Rebaudioside) und/oder ausgeprägte zusätzliche Aromaeindrücke (z.B. Glycerrhyzinsäureammoniumsalz). Einige der Süßstoffe sind gegenüber Hitze nicht besonders stabil (z.B. Thaumatin, Brazzein, Monellin), sind nicht in allen Anwendungen stabil (z.B. Aspartam) und sind teilweise sehr langanhaltend in ihrer süßen Wirkung (starker süßer Nachgeschmack, z.B. Saccharin, Sucralose).

Eine Möglichkeit - ohne den Einsatz von nicht-nutritiven Süßstoffen - besteht darin, den Zuckergehalt von Nahrungs- und/oder Genussmitteln zu senken und sensorisch schwach oder nicht wahrnehmbare Stoffe zuzusetzen, die die Süße mittelbar oder unmittelbar verstärken, wie z.B. in WO 2005/041684 beschrieben. Die in WO 2005/041684 beschriebenen Stoffe sind aber ausdrücklich nicht-natürlichen Ursprungs und somit aus toxikologischer Sicht schwieriger zu bewerten als Stoffe natürlicher Herkunft, insbesondere wenn letztere in Nahrungs- oder Genussmitteln vorkommen oder aus Rohstoffen zur Nahrungs- oder Genussmittelgewinnung stammen. In EP 1 291 342 werden solche Stoffe natürlicher Herkunft (Pyridinium-Betaine) beschrieben; allerdings wird durch sie nicht selektiv der süße Geschmack, sondern auch andere Geschmacksrichtungen wie Umami oder Salzigkeit beeinflusst. Zudem sind die offenbarten Stoffe nur mit hohem Aufwand zu reinigen.

In WO 2007/014879 A1 wird der Einsatz von Hesperetin und in WO 2007/107596 A1 wird Phloretin als Verstärker des süßen Geschmacks von Zucker-reduzierten, der Ernährung oder dem Genuss dienenden Zubereitungen empfohlen. Bisweilen nachteilig ist allerdings beim Einsatz von Hesperetin und Phloretin die vergleichsweise schwach ausgeprägte Süßverstärkung in Nahrungs- und Genussmitteln wie z.B. Joghurt-Produkten, die hohe Anteile Proteine, insbesondere denaturierte Proteine, oder Polysaccharide enthalten. Hesperetin zeigt zudem den Nachteil, in sehr sauren und carbonisierten Anwendungen wie Limonaden oder Cola-Getränken nicht ausreichend wirksam zu sein.

Daher ist es wünschenswert, Stoffe zu finden, die in geringen Konzentrationen süße Geschmackseindrücke von süßen Stoffen, bevorzugt den süßen Geschmackseindruck von Zucker-reduzierten Nahrungs- und Genussmitteln, insbesondere von Zucker-reduzierten Nahrungs- und Genussmitteln mit niedrigem pH-Wert, wirkungsvoll verstärken, ohne das übrige Aromaprofil negativ zu beeinflussen.

Primäre Aufgabe der vorliegenden Erfindung war es, Stoffe anzugeben, die zur Verstärkung, vorzugsweise zur synergistischen Verstärkung, des süßen Geschmackseindrucks eines süß schmeckenden Stoffes geeignet sind, vorzugsweise breit anwendbar sind, vorzugsweise leicht zugänglich sind und bevorzugt natürlich vorkommen. Weiter bevorzugt sollten sich diese Stoffe zudem dazu eignen, einen unangenehmen Geschmackseindrucks, insbesondere einen bitteren Geschmackseindruck eines bitter schmeckenden Stoffes zu maskieren oder zu vermindern.

Insbesondere nicht-nutritive, hochintensive Süßstoffe zeigen oft geschmackliche Probleme (wie oben beschrieben). Insbesondere die natürlich in Stevia ssp. oder Rubus ssp. vorkommenden Steviolglycoside (beispielsweise Steviosid, Rebaudiosid A-H, Dulcoside, Rubusoside, Suavioside A, B und G-J) sind zwar sehr gute Süßstoffe, zeigen aber bei den für eine ausreichende Süßwirkung nötigen Konzentrationen (beispielsweise 400 - 600 ppm für Rebaudiosid A [Reinheit >90 %] in Erfrischungsgetränken, um eine einer 10 %-Konzentration Sucrose entsprechende Süße zu erreichen) schon ausgeprägten Lakritz-artigen und unangenehm bitteren und adstringierenden Neben- und/oder Nachgeschmack.

Insbesondere bei süßen, kalorienfreien oder nahezu kalorienfreien Getränken, die mit Hilfe solcher Süßstoffe hergestellt wurden, senkt dieser unangenehme Neben- und/oder Nachgeschmack häufig die sensorische Akzeptanz und sollte daher maskiert werden.

In der Literatur sind hierfür einige Möglichkeiten beschrieben. So werden in US 2004/0142084 Alkalimetallhydrogensulfate als Maskierungsmittel beschrieben. Diese erhöhen jedoch den Säuregehalt in Anwendungen stark. In US 3,924,017 wurden Kaffeesäurederivate zur Maskierung vorgeschlagen. Nachteilig ist, dass Kaffeesäure selbst leicht bitter schmeckt und leicht die Süße unterdrückt, so dass mehr Süßstoff eingesetzt werden müsste.

In WO 2006/087991 wird der unangenehme Geschmack mit Alkamiden wie Spilanthol unterdrückt; oft ist hier jedoch der kribbelnde Effekt dieser Stoffgruppe nicht erwünscht, so dass diese nicht breit anwendbar sind.

Eine Verbesserung der geschmacklichen Eigenschaften, insbesondere hinsichtlich des Nachgeschmack-Problems von nicht-nutritiven, hochintensiven Süßstoffen kann erreicht werden durch den Einsatz von Tanninsäure, z.B. wie in WO 98/20753 beschrieben, oder Phenolsäuren, z.B. wie in US 3,924,017 beschrieben. Allerdings sind solche Stoffe auf Grund ihrer Catechol-Einheiten nicht besonders stabil in Anwendungen und können als typische Adstringenzien einen bitteren und/oder abstringierenden Neben- und/oder Nachgeschmack verstärken.

Nicht nur die vorangehend genannten Steviolglycoside, sondern auch weitere Stoffe, die einen bitteren Geschmack bzw. Nachgeschmack aufweisen, können in Nahrungs- oder Genussmitteln deren Wert stark mindern (z.B. Flavonoidglycoside und Limonoide in Zitrus-Säften, künstliche Süßstoffe wie Aspartam oder Saccharin, hydrophobe Aminosäuren und/oder Peptide in Käse), auch wenn diese Stoffe gegebenenfalls in Maßen erwünscht und für ein solches Nahrungs- oder Genussmittel charakteristisch sind (z.B. Coffein in Tee oder Kaffee, Chinin in sogenannten Bitter-Lemon-Getränken, Hopfenextrakte in Bier).

Insbesondere zur Senkung des natürlichen Gehalts an Bitterstoffen ist daher oft eine nachträgliche Behandlung nötig, beispielsweise extraktiv wie bei der Entcoffeinierung von Tee bzw. Kaffee, oder enzymatisch, z.B. Behandlung von Orangensaft mit einer Glycosidase zur Zerstörung des bitteren Naringins oder Einsatz von speziellen Peptidasen bei der Reifung von Käse. Diese Behandlung ist belastend für das Produkt, erzeugt Abfallstoffe und bedingt z.B. auch Lösungsmittelreste und andere Rückstände (Enzyme) in den Produkten. Besonders wichtig ist die Unterdrückung eines unangenehmen Geschmackseindrucks, insbesondere eines bitteren Geschmackseindrucks, bei vielen pharmazeutischen Wirkstoffen, da dadurch die Bereitschaft der Patienten, insbesondere bei bitterempfindlichen Patienten wie Kindern, die Zubereitung oral zu sich zu nehmen, deutlich erhöht werden kann. Viele pharmazeutische Wirkstoffe, beispielsweise Aspirin, Salicin, Paracetamol, Ambroxol oder Chinin, um nur eine sehr kleine Auswahl zur Verdeutlichung zu nennen, haben einen ausgeprägten bitteren, adstringierenden und/oder metallischen Geschmack bzw. Nachgeschmack.

Zwar sind bereits einige Stoffe bekannt, die den bitteren Geschmack (zumindest teilweise) unterdrücken können, doch zeigen diese in der Anwendung häufig starke Limitationen (vgl. Ley JP (2008), Masking Bitter Taste by Molecules, Chemosensory Perception, 1(1), 58-77). So wurde Lactisol als Bitter-Maskierer eingesetzt, zeigt aber gleichzeitig einen süßmaskierenden Effekt. Natriumsalze sind beispielsweise auch effektive Bitter-Maskierer, sind aber naturgemäß salzig oder tragen zu einem aus gesundheitlichen Gründen nicht erwünschten Anstieg der Natriumkonzentration im Lebensmittel bei.

Im Zusammenhang mit der vorliegenden Erfindung war es daher ebenfalls wünschenswert, Stoffe zu finden, die (vorzugsweise in geringen Mengen) unangenehme Geschmackseindrücke, insbesondere bittere, adstringierende und/oder metallische Geschmackseindrücke wirkungsvoll unterdrücken oder zumindest vermindern können. Solche Stoffe sollten zudem vorzugsweise breit anwendbar sein, leicht zugänglich sein und natürlich vorkommen.

Im Hinblick auf die vorangehenden Erläuterungen bestand eine besonders bevorzugte Aufgabe der vorliegenden Erfindung darin, Stoffe anzugeben, die sowohl zur synergistischen Verstärkung des süßen Geschmackseindrucks eines süß schmeckenden Stoffes als auch zur Maskierung oder Verminderung eines bitteren Geschmackseindrucks eines bitter schmeckenden Stoffes geeignet sind. Besonders bevorzugt sollten Stoffe angegeben werden, die sowohl den süßen Geschmackseindruck von (z.B. natürlich in Stevia ssp. vorkommenden) Steviolglycosiden synergistisch verstärken als auch den bitteren Geschmackseindruck solcher Steviolglycoside maskieren oder vermindern können.

Eine weitere Aufgabe der vorliegenden Erfindung war es, eine Aromakomposition anzugeben, die den süßen Geschmackseindruck eines süß schmeckenden Stoffes synergistisch verstärken und - sofern der süß schmeckende Stoff einen bitteren Neben- und/oder Nachgeschmacks besitzt - vorzugsweise zudem den bitteren Geschmackseindruck maskieren oder vermindern kann.

Weitere Aufgaben, die der vorliegenden Erfindung zugrunde liegen, ergeben sich aus der nachfolgenden Beschreibung und den beigefügten Patentansprüchen.

Die primäre Aufgabe der vorliegenden Erfindung wird erfindungsgemäß gelöst durch die Verwendung
- einer Verbindung der Formel (I) (d.h. eines Hydroxyflavan-Derivats), 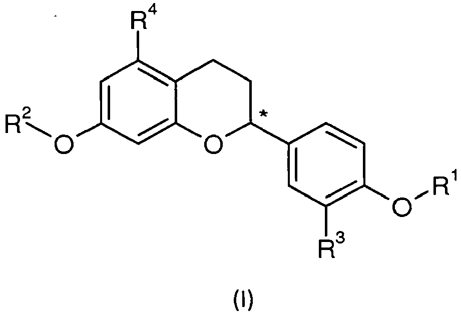 oder
- eines Salzes einer Verbindung der Formel (I)
   oder
- einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
   oder
- eines pflanzlichen Extrakts umfassend eine besagte Verbindung der Formel (I), ein besagtes Salz einer Verbindung der Formel (I) oder eine besagte Mischung
   wobei für die Gruppen R¹, R², R³ und R⁴ sowie die Konfiguration in der Verbindung der Formel (I) bzw. unabhängig voneinander in jeder Verbindung der Formel (I) gilt:
- R¹ und R² bedeuten unabhängig voneinander Wasserstoff, Methyl oder Ethyl,
- R³ und R⁴ bedeuten unabhängig voneinander Wasserstoff, Hydroxy, Methoxy oder Ethoxy und
- die Konfiguration am chiralen Kohlenstoffatom (d.h. an der mit einem "*" markierten Position im obigen Formelbild) ist (R) oder (S),
zur synergistischen Verstärkung des süßen Geschmacks eines süß schmeckenden Stoffes.

Süß schmeckende Stoffe im Sinne der vorliegenden Erfindung sind insbesondere:
- süß schmeckende Kohlenhydrate
   (z.B. Saccharose, Trehalose, Lactose, Maltose, Melizitose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd),
- Zuckeralkohole
   (z.B. Erythritol, Threitol, Arabitol, Ribitol, Xylitol, Sorbitol, Mannitol, Dulcitol, Lactitol),
- Proteine
   (z.B. Miraculin, Pentaidin, Monellin, Thaumatin, Curculin, Brazzein),
- Süßstoffe
   (z.B. Magap, Natriumcyclamat, Acesulfam K, Neohesperidindihydrochalkon, Naringindihydrochalkon, Saccharin, Saccharin-Natriumsalz, Aspartam, Superaspartam, Neotam, Alitam, Sucralose, Lugduname, Carrelame, Sucrononate, Sucrooctate oder natürlich vorkommende Süßstoffe wie Miraculin, Curculin, Monellin, Mabinlin, Thaumatin, Curculin, Brazzein, Pentadin, D-Phenylalanin, D-Tryptophan oder aus natürlichen Quellen gewonnene Extrakte oder Fraktionen, enthaltend solche Aminosäuren bzw. Proteine, Neohesperidindihydrochalkon, Steviolgylcoside, Stevioside, Steviolbiosid, Rebaudioside, Rebaudiosid A, Rebaudiosid B, Rebaudiosid C, Rebaudiosid D, Rebaudiosid E, Rebaudiosid F, Rebaudiosid G, Rebaudiosid H, Dulcoside, Rubusoside, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, Phlomisoside 4, Abrusoside A, Abrusoside B, Abrusoside C, Abrusoside D, Cyclocaryoside A, Cyclocaryoside I, Oslandin, Polypodosid A, Strogin 1, Strogin, 2 Strogin 4, Selligueanin A, Dihydroquercetin-3-acetat, Perillartin, Telosmosid A₁₅, Periandrin I-V, Pterocaryoside, Cyclocaryoside, Mukurozioside, trans-Anethol, trans-Cinnamaldehyd, Bryoside, Bryonoside, Bryonodulcoside, Carnosifloside, Scandenoside, Gypenoside, Trilobatin, Phloridzin, Dihydroflavanole, Hematoxylin, Cyanin, Chlorogensäure, Albiziasaponin, Telosmoside, Gaudichaudiosid, Mogroside, Hernandulcine, Monatin, Glycyrrhetinsäure und deren Derivate und Salze und Phyllodulcin).

Die natürlich vorkommenden Süßstoffe werden im Rahmen einer erfindungsgemäßen Verwendung vorzugsweise in Form von Extrakten oder angereicherten Fraktionen dieser Extrakte als süß schmeckende Stoffe (bzw. gegebenenfalls als sowohl süß als auch unangenehm/bitter schmeckende Stoffe) eingesetzt (vergleiche hierzu die Beschreibung weiter unten), wobei insbesondere Thaumatococcus-Extrakte (Katemfestaude), Extrakte aus *Stevia* ssp. (insbesondere *Stevia rebaudiana*), Swingle-Extrakt (*Momordica* bzw. *Siratia grosvenorii,* Luo-Han-Guo), Extrakte aus *Glycerrhyzia* ssp. (insb. *Glycerrhyzia glabra*), *Rubus* ssp. (insbesondere *Rubus suavissimus*), Citrus-Extrakte, Extrakte aus *Lippia dulcis,* Buddha-Tee-Extrakte (*Hydrangea dulcis* oder *Hydrangea macrophylla*) eingesetzt werden.

Besonders vorteilhaft und daher im Rahmen der vorliegenden Erfindung bevorzugt ist eine wie oben beschriebene Verwendung, wobei die bzw. eine, mehrere oder sämtliche Verbindung(en) der Formel (I) jeweils ausgewählt ist bzw. sind aus der Gruppe bestehend aus den folgenden Verbindungen 1 bis 16:
4',7-Dihydroxyflavan (Verbindung 1),
3',7-Dihydroxy-4'-methoxyflavan (Verbindung 2),
4',7-Dihydroxy-3'-methoxyflavan (Verbindung 3),
4'-Hydroxy-7-methoxyflavan (Verbindung 4),
4',7-Dimethoxy-3'-hydroxyflavan (Verbindung 5),
3',7-Dimethoxy-4'-hydroxyflavan (Verbindung 6),
3',4'-Dihydroxy-7-methoxyflavan (Verbindung 7),
5,7-Dimethoxy-4'-hydroxyflavan (Verbindung 8),
4'-Hydroxy-3',5,7-trimethoxyflavan (Verbindung 9),
3',4'-Dihydroxy-5,7-dimethoxyflavan (Verbindung 10),
4',5-Dihydroxy-3',7-dimethoxyflavan (Verbindung 11),
3',4',5,7-Tetrahydroxyflavan (Verbindung 12),
3',4',5-Trihydroxy-7-methoxyflavan (Verbindung 13),
4',5,7-Trihydroxyflavan (Verbindung 14),
3',5,7-Trihydroxy-4'-methoxyflavan (Verbindung 15),
4',5,7-Trihydroxy-3'-methoxyflavan (Verbindung 16),
wobei unabhängig voneinander in jeder Verbindung 1 bis 16 die Konfiguration am chiralen Kohlenstoffatom (R) oder (S) ist.

Die Strukturen der Verbindungen 1 bis 16 sind zur Verdeutlichung nachfolgend angegeben:

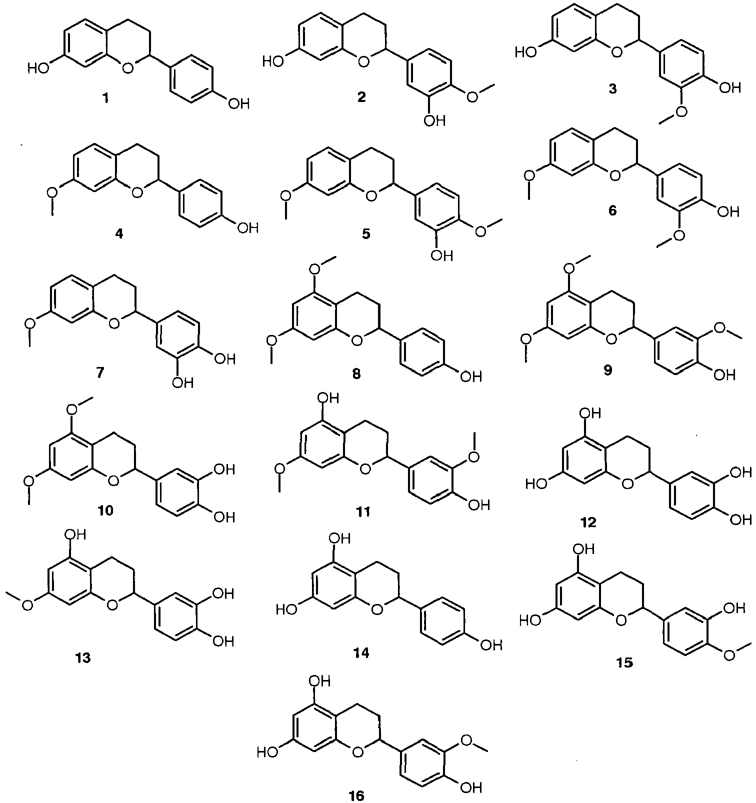

Sofern im Rahmen des vorliegenden Textes bevorzugte bzw. besonders bevorzugte Verbindungen der Formel (I) genannt sind, sind selbstverständlich auch die Salze solcher Verbindungen erfindungsgemäß (besonders) bevorzugt zu verwenden. Weitere Details und bevorzugte Ausgestaltungen erfindungsgemäß zu verwendender Salze werden weiter unten beschrieben.

Die Synthese erfindungsgemäß zu verwendender Verbindungen wird generell z.B. in J. Agric. Food Chem. 1981, 29, 305-312, in Synth. Commun. 2003, Band 33, Seiten 3527-3536 und in Synth. Commun. 1985, Band 15, Seiten 1315-1324 beschrieben.

J. Agric. Food Chem., 1981, 29, 305-312 beschreibt beispielsweise im Rahmen von Untersuchungen zu den Struktur-Wirkungsbeziehungen bestimmter Flavane und Flavanone die erfindungsgemäß zu verwendenden Verbindungen 2 und 15 (dort als Verbindungen 28a und 29a bezeichnet). Dabei wird beschrieben, dass diese einen primären süßen Geschmack und in höheren Konzentrationen gleichzeitig einen bitteren und/oder metallischen Nebengeschmack zeigten. Insbesondere die Existenz einer 3-Hydroxy-4-methoxyphenylgruppe schien im Rahmen der Untersuchungen von Bedeutung zu sein, die für die Verwendung im Rahmen der vorliegenden Erfindung übrigens nicht zwingend notwendig ist.

Dieser Veröffentlichung ist kein Hinweis darauf zu entnehmen, dass eine der dort offenbarten Verbindungen den süßen Geschmackseindruck eines süß schmeckenden Stoffes in synergistischer Weise verstärken kann. Im Übrigen wird auch nicht offenbart, dass eine erfindungsgemäß zu verwendende Verbindung der Formel (I) geschmacksmodulierende, insbesondere maskierende und/oder Geschmacks-vermindernde Wirkungen (gegenüber einem unangenehmen Geschmackseindruck) haben kann. Für Aspekte im Rahmen der vorliegenden Erfindung, die das Maskieren oder Vermindern von unangenehmen Geschmackseindrücken betreffen, siehe unten.

Die erfindungsgemäß zu verwendenden Verbindungen der Formel (I) kommen teilweise in diversen natürlichen Quellen vor. Die folgende Tabelle zeigt (unter Nennung der jeweiligen Literaturstelle) Pflanzen, die anscheinend eine oder mehrere der erfindungsgemäß zu verwendenden Verbindungen der Formel (I) enthalten. Verbindungen der Formel (I), die in der folgenden Tabelle aufgeführt sind, sind im Rahmen der vorliegenden Erfindung besonders bevorzugt zu verwenden.

| **Quelle / Pflanze** | **Verbindung(en) der Formel (I)** | **Literatur** |
|---|---|---|
| *Bauhinia manca* | 1, 3, 4, 6, | Achenbach et al. (1988) Phytochemistry, Band 27, Seiten 1835-1841 |
| *Lycoris aurea* | 1, 2, 4 | Yang et al. (2005) Tianran Chanwu Yanjiu Yu Kaifa, Band 17, Seiten 539-541 |
| *Hippeastrum x hortorum* | 2 | Wink und Lehmann (1996) Botanica Acta, Band 109, Seiten 412-421 |
| *Brosimum acutifolium* | 1, 2 | Takashima und Ohsaki (2001) J. Nat. Prod., Band 64, Seiten 1493-1496 |
| *Knema austrosiamensis* | 3 | Gonzales et al. (1993) Phytochemistry, Band 32, Seiten 433-438 |
| *Dracaena cinnabaria* | 1, 2, 3 | Masaoud et al. (1995) Phytochemistry, Band 38, Seiten 745-749 |
| *Terminalia argenta* | 2, 3 | Garcez et al. (2003) Biochemical Systematics and Ecology, Band 31, 229-232 |
| *Iranthera elliptica* | 3 | Filho et al. (1980) . Phytochemistry, Band 19, Seiten 455-459 |
| *Iranthera grandis* | 3,8 | Diaz und Diaz (1986) Phytochemistry, Band 25, Seiten 2395-2398 |
| *Zephyranthes flava* | 3 | Saini und Ghosal (1984) Phytochemistry, Band 23, Seiten 2415-2422 |
| *Mariscus psilostachys* | 3, 5, 9, 10,11 | Garo et al. (1996) Phytochemistry, Band 43, Seiten 1265-1269 |
| *Cyclopia subternata* (Honigbusch) | 12 | Kamara et al. (2004) J. Agric. Food Chem., Band 52, Seiten 5391-5395 |

Dementsprechend kann eine bzw. können mehrere oder sämtliche der erfindungsgemäß zu verwendenden Verbindungen der Formel (I) auch in Form von pflanzlichen Extrakten, insbesondere in Form von pflanzlichen Extrakten aus einer in der vorangehenden Tabelle genannten Pflanzen, verwendet werden. Die Gewinnung und erfindungsgemäße Verwendungen pflanzlicher Extrakte werden weiter unten beschrieben.

Besonders bevorzugt besitzt bzw. besitzen eine, mehrere oder sämtliche erfindungsgemäß zu verwendende Verbindung(en) der Formel (I) jeweils wenigstens eine, vorzugsweise drei, zwei, oder eine Hydroxy-Gruppe(n).

Weiter bevorzugt ist eine Verwendung (wie oben beschrieben), wobei für die Gruppen R² und R⁴ in der Verbindung der Formel (I) bzw. unabhängig voneinander in einer, mehreren oder sämtlichen, vorzugsweise sämtlichen, Verbindung(en) der Formel (I) gilt:
- R² und/oder R⁴, vorzugsweise R² und R⁴, bedeuten Wasserstoff.

Besonders bevorzugt gilt dabei für die Gruppen R¹ und R³ in der Verbindung der Formel (I) bzw. unabhängig voneinander in einer, mehreren oder sämtlichen, vorzugsweise sämtlichen, Verbindung(en) der Formel (I):
- R¹ bedeutet Wasserstoff oder Methyl
   und/oder
- R³ bedeutet Wasserstoff oder Hydroxy.

Ganz besonders bevorzugt ist eine erfindungsgemäße Verwendung (wie oben beschrieben), wobei für die bzw. eine, mehrere oder sämtliche Verbindungen der Formel (I) gilt:
- R² und R⁴ bedeuten Wasserstoff,
- R¹ bedeutet Wasserstoff oder Methyl und
- R³ bedeutet Wasserstoff oder Hydroxy.

Eine besonders starke synergistische Verstärkung des süßen Geschmacks eines süß schmeckenden Stoffes im Sinne der vorliegenden Erfindung lässt nach eigenen Untersuchungen insbesondere durch eine erfindungsgemäße Verwendung (wie oben beschrieben) erreichen, wobei die bzw. eine oder zwei Verbindung(en) der Formel (I) ausgewählt ist bzw. sind aus der Gruppe bestehend aus 4',7-Dihydroxyflavan (Verbindung 1) und 3',7-Dihydroxy-4'-methoxyflavan (Verbindung 2), wobei unabhängig voneinander in jeder Verbindung 1 bzw. 2 die Konfiguration am chiralen Kohlenstoffatom (R) oder (S) ist.

Die Verbindungen 1 und 2 der Formel (I), insbesondere aber Verbindung 2, eignen sich vorteilhafterweise besonders gut zur synergistischen Verstärkung des süßen Geschmackseindrucks eines süß schmeckenden Stoffes. Zudem sind die besonders bevorzugt zu verwendenden Verbindungen 1 und 2 vorteilhafterweise breit anwendbar, leicht zugänglich und kommen in natürlichen Quellen vor (wie oben beschrieben). Eine besonders vorteilhafte, synergistisch süß verstärkende Wirkung dieser Verbindungen, insbesondere von Verbindung 2, ergibt sich, wenn der süß schmeckende Stoff, dessen süßer Geschmackseindruck erfindungsgemäß synergistisch zu verstärken ist, ein Zucker, insbesondere Sucrose, Glucose oder Fructose oder eine Kombination zweier oder sämtlicher dieser Zucker ist. Die besonders vorteilhafte synergistisch süß verstärkende Wirkung von Verbindung 2 wird weiter unten exemplarisch anhand der Verstärkung des süßen Geschmackeindrucks von Sucrose gezeigt (siehe Anwendungsbeispiel 2).

Insbesondere Verbindung 2 eignet sich überraschenderweise zudem dazu, einen unangenehmen Geschmackseindrucks, insbesondere einen bitteren Geschmackseindruck eines bitter schmeckenden Stoffes zu maskieren oder zu vermindern. Details und weitere Aspekte betreffend das Maskieren oder Vermindern von unangenehmen Geschmackseindrücken sind weiter unten beschrieben.

Zudem bevorzugt ist die erfindungsgemäße Verwendung einer Mischung aus zwei unterschiedlichen Verbindungen der Formel (I) (wie oben beschrieben), vorzugsweise aus zwei unterschiedlichen vorangehend als besonders bevorzugt bezeichneten Verbindungen der Formel (I),
wobei
- die Gruppen R¹ bis R⁴ der beiden Verbindungen identisch sind und
- die Konfiguration am chiralen Kohlenstoffatom der einen Verbindung (R) ist und die Konfiguration am chiralen Kohlenstoffatom der anderen Verbindung (S) ist.

Eine solche erfindungsgemäß zu verwendende Mischung ist entweder eine racemische oder eine nicht racemische Mischung, vorzugsweise aber eine racemische Mischung.

Ganz besonders bevorzugt ist jedoch eine erfindungsgemäße Verwendung (wie oben beschrieben), wobei die bzw. eine Verbindung der Formel (I) (S)-3',7-Dihydroxy-4'-methoxyflavan (Verbindung S-2) ist. Die Struktur der Verbindung S-2 ist zur Verdeutlichung nachfolgend angegeben:

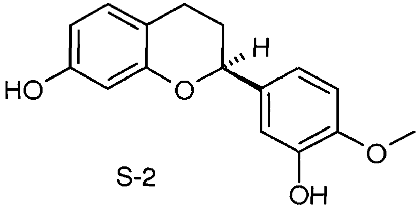

In einer weiteren Ausgestaltung der erfindungsgemäßen Verwendung (wie oben beschrieben) ist es bevorzugt, dass neben (S)-3',7-Dihydroxy-4'-methoxyflavan (Verbindung S-2) kein (R)-3',7-Dihydroxy-4'-methoxyflavan oder eine gegenüber der Menge an (S)-3',7-Dihydroxy-4'-methoxyflavan (Verbindung S-2) geringere Menge an (R)-3',7-Dihydroxy-4'-methoxyflavan vorliegt.

Bei erfindungsgemäß zu verwendenden Salzen einer Verbindung der Formel (I) gilt hinsichtlich der bevorzugten Bedeutungen der Reste das weiter oben Gesagte entsprechend, wobei jedoch eine, mehrere oder sämtliche Hydroxy-Gruppen der Verbindung der Formel (I) (sofern vorhanden) deprotoniert ist bzw. sind. Neben der bzw. den (deprotonierten) Verbindung(en) der Formel (I) liegt dabei eine entsprechende Menge an Gegenkationen vor, wobei diese vorzugsweise ausgewählt sind aus der Gruppe bestehend aus: einfach positiv geladene Kationen der ersten Haupt- und Nebengruppe, Ammoniumionen, Trialkylammoniumionen, zweifach positiv geladene Kationen der zweiten Haupt- und Nebengruppe sowie dreifach positiv geladene Kationen der dritten Haupt- und Nebengruppe, sowie Mischungen davon. Der maximale Deprotonierungsgrad einer einem solchen Salz zugrunde liegenden Verbindung der Formel (I) ergibt sich aus der Zahl an Hydroxy-Gruppen dieser Verbindung. Aus der Zahl an deprotonierten Hydroxy-Gruppen ergibt sich wiederum die entsprechende Menge an Gegenkationen (in Abhängigkeit von ihrer Ladung). So gilt beispielsweise für eine einem solchen Salz zugrunde liegende Verbindung der Formel (I) mit zwei Hydroxy-Gruppen, dass bei vollständiger Deprotonierung der Hydroxy-Gruppen ein zweifach negativ geladenes Anion vorliegt, woraus sich wiederum die Zahl an positiven Ladungen ergibt (hier: zwei), die durch das bzw. die Gegenkation(en) bereitgestellt werden muss. Besonders bevorzugt handelt es sich bei diesen Gegenkationen um Kationen ausgewählt aus der Gruppe bestehend aus Na⁺, K⁺, NH₄⁺, Ca²⁺, Mg²⁺, Al³⁺ und Zn²⁺.

Dementsprechend besonders bevorzugt ist die erfindungsgemäße Verwendung eines Salzes oder einer Mischung aus
- zwei oder mehr unterschiedlichen Salzen von Verbindungen der Formel (I), vorzugsweise von vorangehend als bevorzugt bezeichneten Verbindungen der Formel (I),
   oder
- einer oder mehreren unterschiedlichen Verbindungen der Formel (I) und einem oder mehreren unterschiedlichen Salzen von Verbindungen der Formel (I),
wie oben beschrieben,
wobei das bzw. die Gegenkation(en) des bzw. eines, mehrerer oder sämtlicher der Salze von Verbindungen der Formel (I) ausgewählt ist bzw. sind aus der Gruppe bestehend aus Na⁺, K⁺, NH₄⁺, Ca²⁺, Mg²⁺, Al³⁺ und Zn²⁺.

Für die dem bzw. den Salzen zugrundeliegende(n) Verbindung(en) der Formel (I) gilt das vorangehend Gesagte entsprechend.

Bei eigenen Untersuchungen im Rahmen der vorliegenden Erfindung wurde überraschenderweise gefunden, dass die erfindungsgemäß zu verwendenden Verbindungen der Formel (I) (vorteilhafterweise auch bereits in sehr geringen Konzentrationen) den unangenehmen Geschmackseindruck, insbesondere den bitteren Geschmackseindruck einer Vielzahl von unangenehmen bzw. bitter schmeckenden Stoffen (im Idealfall vollständig) maskieren oder (zumindest) vermindern können, insbesondere von Methylxanthinen wie z.B. Coffein, Alkaloiden wie z.B. Chinin, Flavonoiden wie z.B. Catechinen, Naringin, Neohesperidin, Phenolglycosiden wie z.B. Salicin, Arbutin, Amygdalin oder Phenolen wie z.B. Hydroxytyrosol oder Oleuropein, anorganischen Salzen wie z.B. Kaliumchlorid oder Magnesiumsulfat, pharmazeutischen Wirkstoffen wie z.B. Denatoniumbenzoat oder beta-Lactamantibiotika, Steviolglycosiden wie z.B. Steviosid oder Rebaudiosiden.

Dabei ist es besonders vorteilhaft, dass die erfindungsgemäß zu verwendenden Verbindungen der Formel (I) keine komplexierenden Eigenschaften zeigen. Die genannten Vorteile treffen in erhöhtem Maße für die besonders bevorzugt zu verwendende Verbindung 2 (wie oben beschrieben) zu. Die Verbindung 2 eignet sich demnach vorteilhafterweise besonders gut, um sowohl einen angenehmen Geschmackseindruck, insbesondere den süßen Geschmackseindruck eines süß schmeckenden Stoffes, in synergistischer Weise zu verstärken, als auch, um einen unangenehmen Geschmackseindruck, insbesondere den bitteren Geschmackseindruck eines bitter schmeckenden Stoffes zu maskieren oder vermindern.

Ein weiterer Aspekt, der im Zusammenhang mit der vorliegenden Erfindung steht, betrifft daher die Verwendung
- einer Verbindung der Formel (I)
   oder
- eines Salzes einer Verbindung der Formel (I)
   oder
- einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
   wie jeweils oben beschrieben,
   in einer Mischung umfassend
- einen unangenehm schmeckenden Stoff (B)
   oder
- einen sowohl süß als auch unangenehm schmeckenden Stoff (C),
   (a) zur synergistischen Verstärkung des süßen Geschmacks des Stoffes (C) und/oder
   (b) zum Maskieren oder Vermindern des unangenehmen Geschmackseindrucks des unangenehm schmeckenden Stoffes (B) bzw. (C).

Das vorangehend für die bevorzugten Verbindungen der Formel (I), deren Salze bzw. Mischungen davon sowie für die süß schmeckenden Stoffe Gesagte gilt hierbei entsprechend.

Eine besondere, oben genannte Aufgabe der vorliegenden Erfindung wird gelöst durch eine erfindungsgemäße Verwendung (wie oben beschrieben), in einer Mischung umfassend
- einen süß schmeckenden Stoff (A) und einen unangenehm schmeckenden Stoff (B)
   oder
- einen sowohl süß als auch unangenehm schmeckenden Stoff (C),
   (a) zur synergistischen Verstärkung des süßen Geschmacks des süß schmeckenden Stoffes (A) bzw. (C)
      und
   (b) zum Maskieren oder Vermindern des unangenehmen Geschmackseindrucks des unangenehm schmeckenden Stoffes (B) bzw. (C).

Besonders bevorzugt ist dabei der unangenehme Geschmackseindruck ein bitterer Geschmackseindruck und somit der unangenehm schmeckende Stoff (B) bzw. der sowohl süß als auch unangenehm schmeckende Stoff (C) insbesondere ein bitter schmeckender Stoff (B) bzw. ein sowohl süß als auch bitter schmeckender Stoff (C).

Unter einer Mischung im Rahmen des Ausdrucks "Verwendung (...) in einer Mischung" wird im Rahmen des vorliegenden Textes vorzugsweise eine zur oralen Aufnahme bestimmte pharmazeutische, eine kosmetischen oder eine der Ernährung, der Mundpflege oder dem Genuss dienende Zubereitung, vorzugsweise eine erfindungsgemäße Zubereitung (wie unten beschrieben) verstanden.

Süß schmeckende Stoffe (A) sind vorzugsweise süß schmeckende Stoffe wie oben beschrieben, d.h. insbesondere süß schmeckende Kohlenhydrate, Zuckeralkohole, Proteine oder Süßstoffe (wie jeweils oben beschrieben).

Die süß schmeckenden Stoffe (A) können neben dem süßen Primärgeschmack einen oder mehrere weitere Geschmackseindrücke (und/oder Geruchseindrücke), insbesondere einen nicht süßen Nachgeschmackseindruck, besitzen. Als Primärgeschmack ist dabei der Geschmackseindruck zu verstehen, der auftritt, während ein Stoff sich in direktem Kontakt mit den Schleimhäuten der Mundhöhle, insbesondere mit der Zunge befindet (in der Regel einige Sekunden bis Minuten andauernd). Als Nachgeschmack ist insbesondere der Geschmackseindruck zu verstehen, der nach Leeren der Mundhöhle durch Schlucken und/oder Ausspucken durch Anhaften von Restmengen des Stoffes bestehen bleibt und mehrere Minuten bis Stunden anhalten kann.

Insbesondere können süß schmeckende Stoffe, wie einleitend erwähnt, zusätzliche unangenehme, insbesondere bittere, Geschmackseindrücke besitzen. Solche sowohl süß als auch unangenehm bzw. bitter schmeckende Stoffe sind bevorzugt einzusetzende sowohl süß als auch unangenehm schmeckende Stoffe (C) im Sinne der vorliegenden Erfindung. Das Vorhandensein weiterer Geschmackseindrücke eines Stoffes, aber auch die Intensität des Primärgeschmacks selbst, kann beispielsweise in Abhängigkeit der Konzentration des Stoffes, der Temperatur, des pH-Werts und/oder der weiteren neben diesem Stoff vorliegenden Stoffe variieren.

So gilt beispielsweise für den Umgang mit Steviosid oder Rebaudiosid A oder einem anderen Steviolglycosid (wie oben beschrieben), dass die Süßkraft desselben von diversen Faktoren wie Temperatur, pH-Wert, Konzentration und dem zu süßenden Produkt abhängt. Insbesondere in Abhängigkeit von der Konzentration tritt (bei zu hohen Konzentrationen, insbesondere bei mehr als 50 ppm, insbesondere bei 50 bis 2000 ppm, ganz besonders bei 100 ppm bis 1000 ppm) ein bitterer Beigeschmack auf, der in der Regel unerwünscht ist. Steviosid oder Rebaudiosid A sind im Rahmen der vorliegenden Erfindung besonders bevorzugte süß als auch bitter schmeckende Stoffe (C).

Die unangenehm bzw. bitter schmeckenden Stoffe (B) können neben einem unangenehmen Geschmack ebenfalls weitere, (häufig nicht unangenehme) Geschmacks- und/oder Geruchsqualitäten besitzen. Als weitere, im Sinne der vorliegenden Erfindung nicht unangenehme Geschmacksqualitäten sind z.B. die Eindrücke würzig, umami, süß, salzig, sauer, scharf, kühlend, wärmend, brennend oder kribbelnd zu nennen.

Unangenehm bzw. bitter schmeckende Stoffe mit zusätzlichem süßen Geschmackseindruck sind im Sinne der vorliegenden Erfindung wiederum den sowohl süß als auch unangenehm schmeckenden Stoffen (C) zuzuordnen.

Folglich ist ein sowohl süß als auch unangenehm bzw. bitter schmeckender Stoff (C) vorzugsweise ein Stoff ausgewählt aus der Gruppe süß schmeckender Stoffe wie oben beschrieben oder aus der Gruppe unangenehm schmeckender Stoffe wie unten beschrieben.

Im Rahmen der vorliegenden Erfindung sind den jeweiligen unangenehmen Geschmackseindrücken gleichermaßen entsprechende, durch einen Nachgeschmack bedingte unangenehme Geschmackseindrücke zu- bzw. unterzuordnen.

Unangenehm schmeckende Stoffe im Sinne der vorliegenden Erfindung sind daher:
- Stoffe, die bitter, adstringierend, pappig, kalkig, staubig, trocken, mehlig, ranzig und/oder metallisch schmecken sowie
- Stoffe, die einen entsprechenden (stark anhaltenden) Nachgeschmack haben.

Ein bitterer Geschmackseindruck geht dabei oft einher mit den Geschmackseindrücken adstringierend, pappig, kalkig, staubig, trocken, mehlig, ranzig und/oder metallisch.

Stoffe, die bitter, adstringierend, pappig, kalkig, staubig, trocken, mehlig, ranzig oder metallisch schmecken, sind beispielsweise:
Xanthinalkaloide, Xanthine (Coffein, Theobromin, Theophyllin), Alkaloide (Chinin, Brucin, Strychnin, Nicotin), phenolische Glycoside (z.B. Salicin, Arbutin), Flavonoidglycoside (z.B. Hesperidin, Naringin), Chalcone oder Chalconglycoside, hydrolisierbare Tannine (Gallus- oder Elagsäureester von Kohlenhydraten, z.B. Pentagalloylglucose), nichthydrolisierbare Tannine (ggfs. galloylierte Catechine oder Epicatechine und deren Oligomeren, z.B. Proanthyocyanidine oder Procyanidine, Thearubigenin), Flavone (z.B. Quercetin, Taxifolin, Myricetin), andere Polyphenole (gamma-Oryzanol, Kaffeesäure oder deren Ester), terpenoide Bitterstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, beta-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat oder andere Denatoniumsalze, Sucraloseoctaacetat, Harnstoff, ungesättigte Fettsäuren, insbesondere ungesättigte Fettsäuren in Emulsionen, Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus).

Stoffe, insbesondere Aroma- oder Geschmackstoffe, weisen häufig einen bitteren, adstringierenden, pappigen, kalkigen, staubigen, trockenen, mehligen, ranzigen und/oder metallischen Nachgeschmack auf, obwohl sie einen nicht unangenehmen (Primär-) Geschmack im Sinne der obigen Definition (d.h. z.B. süß, salzig, würzig, sauer, etc.) und/oder Geruch besitzen. Diese einen unangenehmen (Nach-)Geschmack aufweisenden Aroma- oder Geschmackstoffe sind unangenehm schmeckende Stoffe oder insbesondere (sofern sie einen süßen (Primär-)Geschmack besitzen) sowohl süß als auch unangenehm schmeckende Stoffe im Sinne der vorliegenden Erfindung. Diese Aroma- oder Geschmackstoffe sind vorzugsweise ausgewählt aus der Gruppe der Süßstoffe (wie oben beschrieben) oder Zuckeraustauschstoffe, d.h. vorzugsweise besitzen diese Aroma- oder Geschmackstoffe einen süßen (Primär-)Geschmack. Konkrete Beispiele für solche Aroma- oder Geschmackstoffe sind Aspartam, Neotam, Superaspartam, Saccharin, Sucralose, Tagatose, Monellin, Stevioside, Rebaudioside, vor allem Rebaudiosid A, Mogroside, insbesondere Mogrosid V, Thaumatin, Miraculin, Glycyrrhizin, Glycyrrhetinsäure oder deren Derivate, Cyclamat oder die pharmazeutisch akzeptablen Salze der vorgenannten Verbindungen. Diese Aroma- oder Geschmackstoffe sind für erfindungsgemäße Verwendungen bevorzugt einzusetzende sowohl süß als auch unangenehm/bitter schmeckende Stoffe (C). Der unangenehme Geschmackseindruck dieser Stoffe, insbesondere ein bitterer Geschmackseindruck dieser Stoffe, wird bei einer erfindungsgemäßen Verwendung (wie oben beschrieben) besonders effektiv maskiert bzw. vermindert.

Weitere (unangenehm oder sowohl süß als auch unangenehm schmeckende) Stoffe, deren unangenehmer (Neben-)Geschmack erfindungsgemäß vorteilhafterweise maskiert oder vermindert werden kann, sind beispielsweise Aromastoffe, die einen süßen Geruchseindruck verursachen und vorzugsweise ausgewählt sind aus der Gruppe bestehend aus: Vanillin, Ethylvanillin, 2-Hydrox-4-methoxybenzaldehyd, Ethylvanillinisobutyrat (= 3-Ethoxy-4-isobutyryloxybenzaldehyd), Furaneol^{®} (2,5-Dimethyl-4-hydroxy-3(2H)-furanon) und Abkömmlinge (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Abkömmlinge (z.B. Ethylmaltol), Cumarin und Abkömmlinge, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd.

Besonders bevorzugt ist eine erfindungsgemäße Verwendung (wie oben beschrieben), wobei der bitter schmeckende Stoff (B) oder der sowohl süß als auch bitter schmeckende Stoff (C), insbesondere der sowohl süß als auch bitter schmeckende Stoff (C), ausgewählt ist aus der Gruppe bestehend aus Steviolglycosiden, insbesondere aus Steviosid und Rebaudiosiden. Vorzugsweise ist der bitter schmeckende Stoff (B) bzw. der sowohl süß als auch bitter schmeckende Stoff (C) ausgewählt ist aus der Gruppe bestehend aus Rebaudiosid A, Rubusosid, Dulcoside, Mogroside, Phyllodulcin, Glycyrrhetinsäure oder Extrakte aus Stevia ssp. (insbesondere Stevia rebaudiana), Luo Han Guo, Rubus suavissimus, Hydrangea dulcis oder Glycyrrhyza glabra.

Durch die erfindungsgemäße Verwendung synergistisch süß-verstärkender Verbindungen der Formel (I) bzw. deren Salze oder Mischungen davon kann der Gesamtgehalt an süß schmeckenden Stoffen (z.B. in der Ernährung oder dem Genuss dienenden Zubereitungen) vorteilhafterweise reduziert werden, ohne den süßen Geschmackseindruck insgesamt zu vermindern. Dies ist nicht nur aus gesundheitlichen Gründen, sondern auch hinsichtlich der geschmacklichen Eigenschaften von Bedeutung. Ein süß (und bei hohen Konzentrationen auch bitter) schmeckender Stoff wie zum Beispiel Steviosid kann nämlich in Kombination mit erfindungsgemäß zu verwendenden Verbindungen der Formel (I) bzw. deren Salzen oder Mischungen davon vorteilhafterweise (unter Beibehaltung des süßen Geschmackseindrucks) in so geringen Konzentrationen eingesetzt werden, dass kein oder zumindest nur ein verminderter bitterer (Bei-)Geschmack des sowohl süß als auch bitter schmeckenden Stoffes bzw. von Steviosid wahrgenommen wird. Zudem kann der bittere Geschmackseindruck (des sowohl süß als auch bitter schmeckenden Stoffes) durch eine erfindungsgemäß bevorzugte Verwendung (wie oben beschrieben) vorteilhafterweise maskiert oder zumindest (weiter) vermindert werden.

Verschiedene Studien mit Stevia-Extrakten (Yamada A. et al. (1985): Chronic toxicity study of dietary Stevia extracts in F344 rats. In: J. Food Hyg. Soc. Japan. Bd. 26, S. 169-183; Melis, M.S. (1999): Effects of chronic administration of Stevia rebaudiana on fertility in rats. In: J. Ethnopharmacol. Bd. 67, S. 157-161) berichteten über Effekte auf das männliche Reproduktionssystem, wie zum Beispiel reduzierte Spermatogenese, vermindertes Gewicht der Samenbläschen und interstitielle Zellwucherung in den Hoden. Zudem ist bekannt, dass die Blätter von Stevia rebaudiana von paraguayanischen Indianern in Tee als Kontrazeptivum für Männer benutzt worden sind.

Durch die synergistisch süß-verstärkende Wirkung der erfindungsgemäß zu verwendenden Verbindungen der Formel (I) bzw. deren Salzen, insbesondere durch die synergistisch süß-verstärkende Wirkung von Verbindung 2 (wie oben beschrieben), kann die für einen bestimmten Süßegrad benötigte Menge an Stevia-Extrakt bzw. Steviolglycosiden, insbesondere an Steviosid, durch Kombination mit einer oder mehreren Verbindungen der Formel (I) bzw. Salzen davon (unter Beibehaltung des gewünschten Süßegrads) vorteilhafterweise reduziert werden, so dass (gegebenenfalls vorhandene) nachteilige Wirkungen (wie oben beschrieben) vermindert bzw. vermieden werden können.

Zudem kann eine Reduzierung der benötigten Menge an Stevia-Extrakt bzw. Steviolglycosiden neben den bereits genannten Vorteilen auch Kostenersparnisse mit sich bringen.

Wie bereits erwähnt, kann eine bzw. können mehrere oder sämtliche der erfindungsgemäß zu verwendenden Verbindungen der Formel (I) auch in Form von pflanzlichen Extrakten verwendet werden.

Die pflanzlichen Extrakte werden hierfür vorzugsweise aus den entsprechenden frischen oder getrockneten Pflanzen oder Pflanzenteilen gewonnen. Üblicherweise werden die getrockneten Pflanzenteile (z.B. frische oder getrocknete Wurzeln, Wurzelrinde, Knollen, Zwiebeln, andere unter- oder oberirdische Speicherorgane, Scheinfrüchte, Früchte, Samen, Rinde, Holz, Mark, Bast, Stängel, Stiele, Blätter oder Blüten[teile]), vorzugsweise in zerkleinerter Form, mit einem für Nahrungs- und Genussmittel geeigneten Lösungsmittel bei Temperaturen von 0°C bis zum Siedepunkt des jeweiligen Lösungsmittels oder Lösungsmittelgemisches extrahiert, anschließend filtriert und das Filtrat ganz oder teilweise eingeengt, vorzugsweise durch Destillation, Gefrier- oder Sprühtrocknung. Der so erhaltene Rohextrakt kann dann noch weiter aufgearbeitet werden, beispielsweise gegenextrahiert, über Verteilungs-, Absorptions-, Ausschluss-, Affinitäts- oder lonenchromatographie gereinigt, destilliert, sublimiert, mit Adsorptionsmitteln wie Aktivkohlen, Bentonit, Kieselgur etc. gereinigt, enzymatisch (z.B. mit Glycosidasen zur Ausbeutesteigerung an nicht-zuckerhaltigen Molekülen), mit Säure (z.B. unter Druck), mit geeigneten basischen Lösungen z.B. von Hydroxiden, Carbonaten oder Hydrogencarbonaten von Natrium, Kalium, Calcium, Magnesium und Zink, mit sauren lonentauschern oder mit Wasserdampf behandelt werden, in der Regel bei Drücken von 0,01 mbar bis 100 bar, vorzugsweise bei 1 mbar bis 20 bar, mit einem Hilfs- und Trägerstoff versetzt und ggfs. getrocknet (z.B. sprühgetrocknet) und/oder in einem für Nahrungs- und Genussmittel geeigneten Lösungsmittel aufgenommen werden.

Für Nahrungs- und Genussmittel zur Extraktion geeignete Lösungsmittel sind insbesondere Wasser, Ethanol, Methanol, Propylenglycol, Glycerin, Aceton, Dichlormethan, Essigsäureethylester, Diethylether, Hexan, Heptan, Triacetin, pflanzliche Öle oder Fette, superkritisches Kohlendioxid und deren Gemische.

Bevorzugte Hilfs- oder Trägerstoffe sind Maltodextrin, Stärke, natürliche oder künstliche Polysaccharide und/oder Pflanzengummen wie modifizierte Stärken oder Gummi Arabicum, färbende Mittel, z.B. zugelassene Lebensmittelfarbstoffe, färbende Pflanzenextrakte, Stabilisatoren, Konservierungsstoffe, Antioxidantien, viskositäts-beeinflussende Stoffe.

Dementsprechend betrifft die vorliegende Erfindung auch die Verwendung eines pflanzlichen Extrakts umfassend
- eine Verbindung der Formel (I)
   oder
- ein Salz einer Verbindung der Formel (I)
   oder
- eine Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
wie jeweils oben beschrieben,
zur synergistischen Verstärkung des süßen Geschmacks eines süß schmeckenden Stoffes.

Wie bereits beschrieben eignen sich erfindungsgemäß zu verwendende Verbindungen der Formel (I) zudem vorteilhafterweise zum Maskieren oder Vermindern unangenehmer, insbesondere bitterer Geschmackseindrücke.

Demnach betrifft die vorliegende Erfindung insbesondere eine erfindungsgemäße Verwendung eines pflanzlichen Extrakts (wie oben beschrieben) in einer Mischung umfassend
- einen süß schmeckenden Stoff (A) und einen unangenehm, insbesondere einen bitter schmeckenden Stoff (B)
   oder
- einen sowohl süß als auch unangenehm, insbesondere bitter, schmeckenden Stoff (C),
   (a) zur synergistischen Verstärkung des süßen Geschmacks des süß schmeckenden Stoffes (A) bzw. (C)
      und
   (b) zum Maskieren oder Vermindern des unangenehmen Geschmackseindrucks des unangenehm schmeckenden Stoffes bzw. des bitteren Geschmackseindrucks des bitter schmeckenden Stoffes (B) bzw. (C).

Der süß schmeckende Stoff (A) und der bitter schmeckende Stoff (B) bzw. der sowohl süß als auch unangenehm bzw. bitter schmeckende Stoff (C) sind dabei jeweils kein Bestandteil des verwendeten pflanzlichen Extrakts.

Die erfindungsgemäß zu verwendenden Verbindungen der Formel (I) eignen sich im Rahmen erfindungsgemäßer Verwendungen vorteilhafterweise besonders gut für die Verwendung mit Steviolglycosiden.

Dementsprechend ist auch eine wie oben beschriebene erfindungsgemäße Verwendung eines pflanzlichen Extrakts besonders bevorzugt, wobei der bitter schmeckende Stoff (B) oder der sowohl süß als auch bitter schmeckende Stoff (C) ausgewählt ist aus der Gruppe bestehend aus Steviolglycosiden, insbesondere aus Steviosid und Rebaudiosiden, vorzugsweise ausgewählt ist aus der Gruppe bestehend aus Rebaudiosid A, Rubusosid, Dulcoside, Mogroside, Phyllodulcin, Glycyrrhetinsäure oder Extrakte aus Stevia ssp. (insbesondere Stevia rebaudiana), Luo Han Guo, Rubus suavissimus, Hydrangea dulcis oder Glycyrrhyza glabra.

Besonders bevorzugt umfasst ein erfindungsgemäß zu verwendender pflanzlicher Extrakt eine Verbindung 2 (wie oben beschrieben). Im Übrigen gilt das für die bevorzugte Auswahl an Verbindungen der Formel (I) vorangehend Gesagte entsprechend. Auch für die Bedeutung der Stoffe (A), (B) und (C) gilt das vorangehend Gesagte entsprechend.

Bei einer erfindungsgemäßen Verwendung kann es vorteilhaft sein, wenn nicht alle unangenehm bzw. bitter schmeckenden Nuancen (vollständig) maskiert werden, da diese unter Umständen auch erwünscht sein können.

Bei einer erfindungsgemäßen Verwendung (wie oben beschrieben) handelt es sich vorzugsweise um eine Verwendung in einer zur oralen Aufnahme bestimmten pharmazeutischen, einer kosmetischen oder einer der Ernährung, der Mundpflege oder dem Genuss dienenden Zubereitung. Erfindungsgemäße Zubereitungen und deren bevorzugte Ausgestaltungen sind weiter unten beschrieben.

Eine erfindungsgemäß zu verwendende Verbindung der Formel (I) bzw. ein Salz einer solchen Verbindung oder eine Mischung davon (wie oben beschrieben) wird gemäß einem weiteren Aspekt der vorliegenden Erfindung vorzugsweise in einer Aromakomposition zur synergistischen Verstärkung des süßen Geschmacks eines süß schmeckenden Stoffes und/oder zum Maskieren oder Vermindern eines bitteren Geschmackseindrucks eines bitter schmeckenden Stoffes eingesetzt. Eine solche Aromakomposition eignet sich vorteilhafterweise besonders gut zur synergistischen Verstärkung eines süßen und/oder zum Maskieren oder Vermindern eines bitteren Geschmackseindrucks eines sowohl süß als auch bitter schmeckenden Stoffes.

Dementsprechend betrifft die vorliegende Erfindung auch eine Aromakomposition zur synergistischen Verstärkung des süßen Geschmacks eines süß schmeckenden Stoffes (A) und/oder zum Maskieren oder Vermindern eines bitteren Geschmackseindrucks eines bitter schmeckenden Stoffes (B),
vorzugsweise zur synergistischen Verstärkung des süßen Geschmacks und zum Maskieren oder Vermindern des bitteren Geschmackseindrucks eines sowohl süß als auch bitter schmeckenden Stoffes (C),
umfassend oder bestehend aus
(i) - einer Verbindung der Formel (I)
   oder
   - einem Salz einer Verbindung der Formel (I)
      oder
   - einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der
      Formel (I) und einem oder mehreren unterschiedlichen Salzen von Verbindungen der Formel (I),
      oder
   - einem pflanzlichen Extrakts umfassend eine besagte Verbindung der Formel (I), ein besagtes Salz einer Verbindung der Formel (I) oder eine besagte Mischung,
      wie jeweils oben beschrieben,
      sowie
(ii) einem oder mehreren Aroma- und/oder Geschmacksstoffen, wobei diese(r) keine Verbindung der Formel (I) oder ein Salz davon ist bzw. sind und ausgewählt ist bzw. sind aus der Gruppe bestehend aus Aromastoffen, die einen süßen Geruchseindruck verursachen, insbesondere Vanillin, Ethylvanillin, Ethylvanillinisobutyrat (= 3-Ethoxy-4-isobutyryloxybenzaldehyd), Furaneol^{®} (2,5-Dimethyl-4-hydroxy-3(2H)-furanon) und Abkömmlinge bzw. Derivate (z.B. Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und Derivate (z.B. Ethylmaltol), Cumarin und Derivate, gamma-Lactone (z.B. gamma-Undecalacton, gamma-Nonalacton), delta-Lactone (z.B. 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenone, 3-Hydroxy-4,5-dimethyl-2(5H)-furanon, Fruchtester und Fruchtlactone (z.B. Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd ,
   und/oder
(iii) einer oder mehreren (weiteren)
   - Substanz(en) zum Maskieren oder Vermindern eines bitteren Geschmackeindrucks, wobei diese keine Verbindung der Formel (I) oder ein Salz davon ist bzw. sind und ausgewählt ist bzw. sind aus der Gruppe bestehend aus Natriumsalze (z.B. Natriumchlorid, Natriumlactat, Natriumcitrat, Natriumacetat, Natriumgluconoat), Homoeriodictyol oder dessen Natriumsalze, 2,4-Dihydroxybenzoesäurevanillylamid, gamma-Aminobuttersäure, Pellitorin (insbesondere wie in EP 2008530 A1 beschrieben)
      und Gingerdione
      und/oder
   - Substanz(en) zum Verstärken eines süßen Geschmackeindrucks, wobei diese keine Verbindung der Formel (I) oder ein Salz davon ist bzw. sind und vorzugsweise ausgewählt ist bzw.
   sind aus der Gruppe bestehend aus Hesperetin (insbesondere wie in der WO 2007/014879 offenbart), Hydroxyphenylalkadionen (insbesondere wie in WO 2007/003527 beschrieben), Deoxybenzoinen (insbesondere wie in WO 2006/106023 und der deutschen Patentanmeldung mit dem Amtsaktenzeichen 10 2009 002 268.6 beschrieben), 4-Hydroxychalkonen (insbesondere wie in WO 2007/107596 beschrieben), Propenylphenylglycoside (Chavicolglycoside) (insbesondere wie in EP 1 955 601 A1 beschrieben) und Divanillinen (insbesondere wie in WO 2004/078302 beschrieben).

Die vorangehend genannten Dokumente sind bezüglich der darin offenbarten entsprechenden Verbindungen auf dem Wege der Verweisung Bestandteil dieser Anmeldung.

Für bevorzugt enthaltene Verbindungen der Formel (I) bzw. Salze oder Mischungen davon sowie die Stoffe (A), (B) und (C) gilt das weiter oben Gesagte entsprechend.

Die vorliegende Erfindung betrifft auch die Verwendung einer Aromakomposition (wie oben beschrieben) in einer Mischung umfassend
- einen süß schmeckenden Stoff (A) und/oder einen bitter schmeckenden Stoff (B)
   oder
- einen sowohl süß als auch bitter schmeckenden Stoff (C),
   (a) zur synergistischen Verstärkung des süßen Geschmacks des süß schmeckenden Stoffes (A) bzw. (C)
      und/oder
   (b) zum Maskieren oder Vermindern des unangenehmen Geschmackseindrucks des unangenehm schmeckenden Stoffes (B) bzw. (C).

Besonders bevorzugt ist die Verwendung einer Aromakomposition (wie oben beschrieben), wobei der bitter schmeckende Stoff (B) oder der sowohl süß als auch bitter schmeckende

Stoff (C) ausgewählt ist aus der Gruppe bestehend aus Steviolglycosiden, insbesondere aus Steviosid und Rebaudiosiden, vorzugsweise ausgewählt ist aus der Gruppe bestehend aus Rebaudiosid A, Rubusosid, Dulcoside, Mogroside, Phyllodulcin, Glycyrrhetinsäure oder Extrakte aus Stevia ssp. (insbesondere Stevia rebaudiana), Luo Han Guo, Rubus suavissimus, Hydrangea dulcis oder Glycyrrhyza glabra.

Insbesondere betrifft die vorliegende Erfindung auch eine Verwendung einer erfindungsgemäßen Aromakomposition (wie oben beschrieben) zur synergistischen Verstärkung des süßen Geschmacks eines süß schmeckenden Stoffes und/oder zum Maskieren oder Vermindern eines bitteren Geschmackseindrucks eines bitter schmeckenden Stoffes in einer zur oralen Aufnahme bestimmten pharmazeutischen, einer kosmetischen oder einer der Ernährung, der Mundpflege oder dem Genuss dienenden Zubereitung, insbesondere in erfindungsgemäßen Zubereitungen wie unten beschrieben. Besonders bevorzugt wird eine erfindungsgemäße Aromakomposition zur Verbesserung des sensorischen Profils von süßen, oral zu konsumierenden Produkten verwendet.

Die erfindungsgemäß zu verwendenden Verbindungen der Formel (I) bzw. deren Salze oder Mischungen davon oder Aromakompositionen umfassend solche Verbindungen bzw. Salze werden gemäß einem weiteren Aspekt der vorliegenden Erfindung in einer Zubereitung, insbesondere einer der Ernährung, der Mundpflege oder dem Genuss dienenden oder kosmetischen (insbesondere zur Applikation im Bereich des Kopfes) oder in zur oralen Aufnahme bestimmten pharmazeutischen Zubereitungen eingesetzt. Solche Zubereitungen umfassen in der Regel einen oder mehrere süß schmeckende und/oder unangenehm, insbesondere bitter, schmeckende Stoffe oder sowohl süß als auch unangenehm bzw. bitter schmeckende Stoffe (wie jeweils oben beschrieben).

Dementsprechend betrifft die vorliegende Erfindung auch eine Zubereitung, umfassend die Bestandteile
(I) - eine Verbindung der Formel (I),
   oder
   - ein Salz einer Verbindung der Formel (I)
      oder
   - eine Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
      wie jeweils oben beschrieben,
      und
(II) einen oder mehrere süß schmeckende Stoffe (A) und/oder sowohl süß als auch bitter schmeckende Stoffe (C), wobei der bzw. die Stoffe (A) bzw. (C) keine Verbindung der Formel (I) oder ein Salz davon ist bzw. sind,
   wobei die Gesamtmenge an Verbindungen der Formel (I) bzw. Salzen der Verbindung der Formel (I) in der Zubereitung ausreicht, den süßen Geschmackseindruck des bzw. der Stoffe (A) bzw. (C) synergistisch zu verstärken.

Gemäß einer bevorzugten Ausgestaltung der erfindungsgemäßen Zubereitung (wie oben beschrieben) umfasst diese eine erfindungsgemäße Aromakomposition (wie oben beschrieben), die den Bestandteil (I) der Zubereitung, d.h. eine oder mehrere Verbindungen der Formel (I) und/oder Salze davon, enthält.

Für die Stoffe (A) und (C) sowie die Verbindungen der Formel (I), deren Salze und Mischungen davon sowie deren bevorzugte Auswahl gilt jeweils das vorangehend Gesagte entsprechend.

Bevorzugt ist eine erfindungsgemäße Zubereitung (wie oben beschrieben), wobei die Zubereitung eine der Ernährung, der Mundpflege oder dem Genuss dienende oder kosmetische (insbesondere zur Applikation im Bereich des Kopfes) oder zur oralen Aufnahme bestimmte pharmazeutische Zubereitung ist.

Besonders bevorzugt ist eine erfindungsgemäße der Ernährung, der Mundpflege oder dem Genuss dienende oder kosmetische oder eine zur oralen Aufnahme bestimmte pharmazeutische Zubereitung (wie oben beschrieben), wobei die Zubereitung bezogen auf das Gesamtgewicht der Zubereitung 0,0001 Gew.-% (1 ppm) bis 0,5 Gew.-% (5000 ppm), bevorzugt 0,0001 Gew.-% (1 ppm) bis 0,1 Gew.-% (1000 ppm), besonders bevorzugt 0,001 Gew.-% (10 ppm) bis 0,05 Gew.-% (500 ppm), an Bestandteil (I) umfasst.

Erfindungsgemäße Zubereitungen können auch als Halbfertigware oder Würzmischung vorliegen.

Besonders bevorzugt ist eine erfindungsgemäße Zubereitung (wie oben beschrieben), wobei die Zubereitung eine zur Herstellung einer der Mundpflege oder dem Genuss dienenden oder einer kosmetischen Zubereitung oder einer zur oralen Aufnahme bestimmten pharmazeutischen Zubereitung geeignete Halbfertigware ist.

Besonders bevorzugt ist eine Halbfertigware (wie oben beschrieben), wobei die Halbfertigware bezogen auf das Gesamtgewicht der Halbfertigware 0,0001 Gew.-% bis 95 Gew.-%, vorzugsweise 0,001 Gew.-% bis 95 Gew.-%, bevorzugt 0,001 bis 80 Gew.-%, besonders bevorzugt 0,01 Gew.-% bis 50 Gew.-%, an Bestandteil (I) umfasst.

Besonders bevorzugt ist eine erfindungsgemäße Zubereitung (wie oben beschrieben), wobei die Gesamtmenge an Verbindungen der Formel (I) bzw. Salzen der Verbindung der Formel (I) in der Zubereitung ausreicht, um im Vergleich mit einer Zubereitung, die bei ansonsten identischer Zusammensetzung kein(e) Verbindung der Formel (I) bzw. Salz der Verbindung der Formel (I) aber zumindest die 1,05-fache Menge an süß schmeckendem/n Stoff(en) (A) bzw. (C) umfasst, den gleichen oder einen verstärkten Süßeeindruck zu vermitteln.

Ganz besonders bevorzugt ist eine erfindungsgemäße Zubereitung (wie oben beschrieben), wobei Bestandteil (I) eine Verbindung 1 und/oder eine Verbindung 2, vorzugsweise eine Verbindung 2, wie jeweils oben beschrieben, umfasst oder daraus besteht, wobei in jeder Verbindung (unabhängig voneinander) die Konfiguration am chiralen Kohlenstoffatom (R) oder (S) ist. Besonders bevorzugt ist auch eine erfindungsgemäße Zubereitung, umfassend als oder in Bestandteil (I) eine Mischung aus zwei unterschiedlichen Verbindungen der Formel (I), wobei die Gruppen R¹ bis R⁴ der beiden Verbindungen identisch sind und die Konfiguration am chiralen Kohlenstoffatom unterschiedlich ist, d.h. eine Mischung aus zwei Enantiomeren (wie oben beschrieben).

Der Ernährung oder dem Genuss dienende Zubereitungen im Sinne der vorliegenden Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. mit magensaftresistentem Überzug), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Zur oralen Aufnahme bestimmte pharmazeutische Zubereitungen im Sinne der vorliegenden Erfindung sind Zubereitungen, die z.B. in Form von Kapseln, Tabletten (nichtüberzogene sowie überzogene Tabletten, z.B. mit magensaftresistentem Überzug), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Der Mundpflege dienende Zubereitungen im Sinne der vorliegenden Erfindung sind insbesondere Mund- und/oder Zahnpflegemittel wie Zahnpasten, Zahngele, Zahnpulver, Mundwässer, Kaugummis und andere Mundpflegemittel. Besonders bevorzugt sind der Mundpflege dienende Zubereitungen, die einen Extrakt oder Bestandteile eines Extrakts aus Stevia ssp. enthalten. Die Verbindungen der Formel (I), insbesondere die vorangehend als bevorzugt bezeichneten Verbindungen der Formel (I), eignen sich vorteilhafterweise besonders gut, um in der Mundpflege dienenden Steviolglycoside enthaltenden Zubereitungen einen bitteren Geschmackseindruck der Steviolglycoside, insbesondere von Steviosid und/oder Rebaudiosid A zu maskieren oder zu vermindern.

Kosmetische Zubereitungen, insbesondere kosmetische Zubereitungen zur Applikation im Bereich des Kopfes, sind im Rahmen der vorliegenden Erfindung vorzugsweise kosmetische Zubereitungen, die zumindest einen unangenehm, insbesondere einen bitter schmeckenden Stoff (B) beinhalten und selbst bei sachgemäßer Auftragung auf die Haut mit der Mundhöhle in Kontakt treten können. Solche Zubereitungen sind beispielsweise kosmetische Zubereitungen zur Applikation im Bereich des Kopfes wie Seifen, andere Reinigungs- oder Pflegemittel für den Gesichtsbereich, Gesichtscremes, -lotionen oder -salben, Sonnenschutzmittel, Bartreinigungs- oder -pflegemittel, Rasierschäume, -seifen oder -gele, Lippenstifte oder andere Lippenkosmetika oder Lippenpflegemittel.

Vorzugsweise umfasst Bestandteil (II) einer erfindungsgemäßen Zubereitung (wie oben beschrieben)
- einen oder mehrere sowohl süß als auch bitter schmeckende Stoffe (C) (wie oben beschrieben)
   und/oder
- einen oder mehrere bitter schmeckende Stoffe (B), wobei der bzw. die Stoffe (B) keine Verbindung der Formel (I) oder ein Salz davon ist bzw. sind,
   wobei die Gesamtmenge an Verbindungen der Formel (I) bzw. Salzen der Verbindung der Formel (I) in der Zubereitung ausreicht, den bitteren Geschmackseindruck des bzw. der Stoffe (B) bzw. (C) zu maskieren oder vermindern.

Für die Stoffe (B) und deren bevorzugte Auswahl gilt (wie für die Stoffe (A) und (C)) ebenfalls das vorangehend Gesagte entsprechend.

Besonders bevorzugt betrifft die vorliegende Erfindung eine erfindungsgemäße Zubereitung (wie oben beschrieben), wobei Bestandteil (II) einen oder mehrere sowohl süß als auch bitter schmeckende Stoffe (C) umfasst, wobei die Gesamtmenge an Verbindungen der Formel (I) bzw. Salzen der Verbindung der Formel (I) in der Zubereitung ausreicht, sowohl den süßen Geschmackseindruck des bzw. der sowohl süß als auch bitter schmeckenden Stoffe (C) synergistisch zu verstärken als auch den bitteren Geschmackseindruck des bzw. der sowohl süß als auch bitter schmeckenden Stoffe (C) zu maskieren oder vermindern.

Besonders bevorzugt ist eine erfindungsgemäße Zubereitung (wie oben beschrieben), insbesondere eine der Ernährung, dem Genuss oder der Mundpflege dienende Zubereitung, wobei einer, mehrere oder vorzugsweise sämtliche der sowohl süß als auch bitter schmeckenden Stoffe (C) (sofern vorhanden) ausgewählt sind aus der Gruppe bestehend aus Steviolglycosiden, insbesondere aus Steviosid und Rebaudiosid A, vorzugsweise ausgewählt sind aus der Gruppe bestehend aus Rebaudiosid A, Rubusosid, Dulcoside, Mogroside, Phyllodulcin, Glycyrrhetinsäure oder Extrakte aus Stevia ssp. (insbesondere Stevia rebaudiana), Luo Han Guo, Rubus suavissimus, Hydrangea dulcis oder Glycyrrhyza glabra.

Weiter bevorzugt ist eine erfindungsgemäße Zubereitung (wie oben beschrieben), wobei die Gesamtmenge an bitter schmeckenden Stoffen (B) und/oder sowohl süß als auch bitter schmeckenden Stoffen (C) in der Zubereitung ausreicht, um in einer Vergleichszubereitung, die bei ansonsten identischer Zusammensetzung keine Verbindung der Formel (I) bzw. kein Salz der Verbindung der Formel (I) umfasst, als bitterer Geschmack wahrgenommen zu werden, und die Gesamtmenge an Verbindungen der Formel (I) bzw. Salzen der Verbindung der Formel (I) in der Zubereitung ausreicht, um den bitteren Geschmack des bzw. der Stoffe (B) bzw. (C) zu maskieren oder im Vergleich mit der Vergleichszubereitung zu vermindern.

Eine erfindungsgemäße Zubereitung umfasst vorzugsweise zudem zumindest eine weitere Substanz zum Maskieren oder Vermindern eines bitteren, metallischen, kalkigen, sauren oder adstringierenden Geschmackseindrucks und/oder zum Verstärken eines süßen, salzigen oder Umami-Geschmackseindrucks. Bevorzugte weitere Substanzen zum Maskieren oder Vermindern eines unangenehmen Geschmackseindrucks und/oder zum Verstärken eines angenehmen Geschmackseindrucks werden weiter unten beschrieben.

Ganz besonders bevorzugt ist eine erfindungsgemäße Zubereitung (wie oben beschrieben), wobei
- Bestandteil (I) in Form eines pflanzlichen Extrakts (oder einer Fraktion davon), d.h. in Form eines pflanzlichen Extrakts umfassend eine Verbindung der Formel (I), ein Salz einer Verbindung der Formel (I) oder eine Mischung davon (wie oben beschrieben),
   und/oder - Bestandteil (II) in Form eines pflanzlichen Extrakts, vorzugsweise eines Extrakts aus Stevia ssp., insbesondere eines Extrakts aus Stevia rabaudiana, (oder einer Fraktion davon), d.h. in Form eines pflanzlichen Extrakts umfassend einen oder mehrere süß schmeckende Stoffe (A), bitter schmeckende Stoffe (B) und/oder sowohl süß als auch bitter schmeckende Stoffe (C) (wie jeweils oben beschrieben),
   vorliegen. Besonders vorteilhaft ist es, wenn sowohl Bestandteil (I) als auch Bestandteil (II) als pflanzlicher Extrakt (oder Fraktion davon) vorliegen, wobei Bestandteil (II) vorzugsweise in Form eines Extrakts aus Stevia rabaudiana vorliegt. Weitere Details zu pflanzlichen Extrakten umfassend einen oder mehrere Verbindungen der Formel (I) bzw. deren Salze oder Mischungen davon sowie deren Gewinnung wurden weiter oben beschrieben.

Bevorzugt ist auch eine erfindungsgemäße Zubereitung, insbesondere einer der Ernährung, der Mundpflege oder dem Genuss dienende oder kosmetische oder eine zur oralen Aufnahme bestimmte pharmazeutische Zubereitung oder Halbfertigware (wie jeweils oben beschrieben), umfassend eine erfindungsgemäße Aromakomposition (wie oben beschrieben), die wiederum Bestandteil (I) sowie gegebenenfalls Bestandteil (II) der Zubereitung umfasst. Der Anteil einer erfindungsgemäßen Aromakomposition an einer solchen erfindungsgemäßen Zubereitung beträgt vorzugsweise 0,000001 Gew.-% bis 95 Gew.-%, bezogen auf das Gesamtgewicht der Zubereitung.

In einer erfindungsgemäßen Zubereitung können zudem übliche Wirk-, Grund-, Hilfs- und Zusatzstoffe für die Ernährung, der Mundpflege oder dem Genuss dienende oder orale pharmazeutische Zubereitungen (d.h. zur oralen Anwendung bestimmte pharmazeutische Zubereitungen) oder kosmetische Zubereitungen (insbesondere solche zur Applikation im Bereich des Kopfes) in Mengen von 0,9 bis 99,999999 Gew.-%, vorzugsweise 10 bis 80 Gew.-%, bezogen auf das Gesamtgewicht der Zubereitung, enthalten sein. Insbesondere kann eine erfindungsgemäße Zubereitung Wasser in einer Menge bis zu 99,999999 Gew.-%, vorzugsweise 5 bis 80 Gew.-%, bezogen auf das Gesamtgewicht der Zubereitung, enthalten.

Die erfindungsgemäßen Zubereitungen, umfassend eine oder mehrere der erfindungsgemäß zu verwendenden Verbindungen der Formel (I) und/oder Salze von Verbindungen der Formel (I), werden vorzugsweise hergestellt, indem die Verbindung(en) der Formel (I) und/oder das bzw. die Salze einer oder mehrerer solcher Verbindungen mit einem festen oder flüssigen Trägerstoff in Form einer Lösung oder eines Gemisches in eine entsprechende Zubereitung, d.h. insbesondere eine der Ernährung, der Mundpflege oder dem Genuss dienende oder eine zur oralen Anwendung bestimmte pharmazeutische (Basis-) Zubereitung eingearbeitet wird bzw. werden. Als Lösung vorliegende erfindungsgemäße Zubereitungen können vorteilhafterweise auch durch Sprühtrocknung in eine feste Zubereitung überführt werden.

Gemäß einer weiteren bevorzugten Ausführungsform werden zur Herstellung erfindungsgemäßer Zubereitungen die erfindungsgemäß zu verwendenden Verbindungen der Formel (I) und/oder Salze solcher Verbindungen sowie ggf. weitere Bestandteile der erfindungsgemäßen Zubereitung vorher (d.h. vor Einarbeitung in die Zubereitung) in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet.

In einem weiteren bevorzugten Herstellungsverfahren werden die Verbindungen der Formel (I) und/oder Salze davon vorher mit einem oder mehreren geeigneten Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt wird, dass die Verbindung(en) der Formel (I) und/oder das bzw. die Salze einer solchen Verbindung verzögert von der Matrix freigegeben werden, so dass man eine langanhaltende Wirkung erhält.

Als weitere Bestandteile für erfindungsgemäße, der Ernährung oder dem Genuss dienende Zubereitungen können übliche Grund-, Hilfs- und Zusatzstoffe für Nahrungs- oder Genussmittel in einer erfindungsgemäßen Zubereitung enthalten sein (wie oben beschrieben) oder für die Herstellung solcher Zubereitungen verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, weitere Geschmackskorrigenzien bzw. Geschmacksmodulatoren für unangenehme Geschmackseindrücke oder nicht unangenehme Geschmackseindrücke, insbesondere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), (gegebenenfalls weitere) Bitterstoffe (z.B. Chinin, Coffein, Limonin, Amarogentin, Humolone, Lupolone, Catechine, Tannine), (gegebenenfalls weitere) Süßstoffe (z.B. Saccharin, Cyclamat, Aspartam, Neotam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Basis für erfindungsgemäße der Mundpflege dienende Zubereitungen), umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, (ggf. zusätzliche) Süßstoffe, wie z.B. Saccharin, Geschmackskorrigenzien für unangenehme weitere Geschmackskorrigenzien für unangenehme Geschmackseindrücke oder in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für erfindungsgemäße der Mundpflege dienende Zubereitungen), umfassen im allgemeinen eine Kaugummibase, d.h. eine beim Kauen plastisch werdende Kaumasse, Zucker verschiedener Arten, Zuckeraustauschstoffe, Süßstoffe, Zuckeralkoholen, Geschmackskorrigenzien bzw. Geschmacksmodulatoren für unangenehme oder in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Aromen und Stabilisatoren oder Geruchskorrigentien.

Als Bestandteile für erfindungsgemäße orale pharmazeutische Zubereitungen können sämtliche der üblicherweise eingesetzten Wirk-, Grund-, Hilfs- und Zusatzstoffe für zur oralen Anwendung vorgesehene pharmazeutische Zubereitungen verwendet werden. Als Wirkstoffe können insbesondere unangenehm schmeckende oral formulierbare pharmazeutische Wirkstoffe verwendet werden, insbesondere bitter schmeckende Stoffe, deren bitterer Geschmackseindruck erfindungsgemäß maskiert oder vermindert werden kann. Die Wirk-, Grund-, Hilfs- und Zusatzstoffe können in an sich bekannter Weise in die zur oralen Anwendung geeigneten Applikationsformen überführt werden. Dies geschieht regelmäßig unter Verwendung inerter, nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) und Geruchskorrigentien sowie Geschmackskorrigentien, insbesondere solche, die nicht den bitteren Geschmack betreffen.

Bevorzugt können erfindungsgemäße Zubereitungen (wie oben beschrieben) zudem eine (nicht erfindungsgemäße) Aromakomposition enthalten, um den Geschmack und/oder Geruch der Zubereitung (weiter) abzurunden und zu verfeinern. Geeignete Aromakompositionen enthalten z.B. synthetische, natürliche oder naturidentische Aroma-, Riech- und Geschmackstoffe sowie geeignete Hilfs- und Trägerstoffe. Als besonders vorteilhaft wird es dabei angesehen, dass ein bitterer oder metallischer Geschmackseindruck, der von in den erfindungsgemäßen Zubereitungen enthaltenen Aroma- bzw. Riech- oder Geschmackstoffen ausgeht, maskiert oder vermindert werden kann, so dass das gesamte Aroma- oder Geschmacksprofil verbessert wird.

Erfindungsgemäße Zubereitungen, die als Halbfertigwaren vorliegen, können zur Maskierung oder Verminderung eines unangenehmen Geschmackseindrucks von Fertigware-Zubereitungen dienen, die unter Verwendung der Halbfertigware-Zubereitung hergestellt werden.

In einer besonders bevorzugten Ausführung der vorliegenden Erfindung werden die erfindungsgemäß zu verwendenden Verbindungen der Formel (I) bzw. deren Salze in einer erfindungsgemäßen Zubereitungen (wie oben beschrieben) in Kombination mit zumindest einer weiteren Substanz zum Verändern, Maskieren oder Vermindern eines unangenehmen Geschmackseindrucks und/oder zum Verstärken eines angenehmen Geschmackseindrucks verwendet, wobei der angenehme Geschmackseindruck vorzugsweise ein süßer und/oder Umami-Geschmack ist. Auf diese Weise kann ein besonders wirksamer Maskierungseffekt erreicht werden.

Weitere Substanzen zum Maskieren oder Vermindern eines unangenehmen Geschmackseindrucks und/oder zum Verstärken eines angenehmen Geschmackseindrucks bzw. Geschmackskorrigenzien sind - ohne die vorliegende Erfindung darauf zu beschränken - vorzugsweise ausgewählt aus der Gruppe bestehend aus Nucleotide (z.B. Adenosin-5'-monophosphat, Cytidin-5'-monophosphat) oder deren pharmazeutisch akzeptable Salze, Lactisole, Natriumsalze (z.B. Natriumchlorid, Natriumlactat, Natriumcitrat, Natriumacetat, Natriumgluconoat), Hydroxyflavanone, wie zum Beispiel Eriodictyol, Sterubin (Eriodictyol-7-methylether), Homoeriodictyol, und deren Natrium-, Kalium-, Calcium-, Magnesium- oder Zinksalze (insbesondere solche wie beschrieben in EP 1 258 200, die bezüglich der darin offenbarten entsprechenden Verbindungen auf dem Wege der Verweisung Bestandteil dieser Anmeldung wird), Hydroxybenzoesäureamide, wie zum Beispiel 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N*-(4-hydroxy-3-methoxybenzyl)amid, 2,4-Dihydroxybenzoesäure-*N*-(4-hydroxy-3-methoxybenzyl)amid-Mono-natriumsalz, 2,4-Dihydroxybenzoesäure-*N*-2-(4-hydroxy-3-methoxyphenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoine, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023, die bezüglich der darin offenbarten entsprechenden Verbindungen auf dem Wege der Verweisung Bestandteil dieser Anmeldung wird);

Hydroxyphenlaalkandione, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimere (insbesondere solche wie beschrieben in WO 2006/058893, die bezüglich der darin offenbarten entsprechenden Verbindungen auf dem Wege der Verweisung Bestandteil dieser Anmeldung wird); gamma-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird); Divanilline (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalcone (vorzugsweise wie beschrieben in US 2008/0227867 A1, die bezüglich der darin offenbarten entsprechenden Verbindungen auf dem Wege der Verweisung Bestandteil dieser Anmeldung wird), dabei insbesondere Phloretin und Davidigenin, Aminosäuren oder Gemische von Molke-proteinen mit Lecithinen, Hesperitin wie in der WO 2007/014879 offenbart, die hinsichtlich dieser Verbindungen auf dem Wege der Verweisung Bestandteil dieser Anmeldung wird, 4-Hydroxydihydrochalkone wie in der WO 2007/107596 offenbart, die hinsichtlich dieser Verbindungen auf dem Wege der Verweisung Bestandteil dieser Anmeldung wird, oder Propenylphenylglycoside (Chavicolglycoside) wie in EP 1 955 601 A1 beschrieben, die hinsichtlich dieser Verbindungen auf dem Wege der Verweisung Bestandteil dieser Anmeldung wird, Pellitorin und abgeleitete Aromakompositionen wie in US Provisional Application 60/944,854 und den darauf basierenden Patentanmeldungen beschrieben, Umami-Verbindungen wie in WO 2008/046895 A1 und EP 1 989 944 A1 beschrieben, die jeweils hinsichtlich dieser Verbindungen auf dem Wege der Verweisung Bestandteil dieser Anmeldung werden sowie Umami-Verbindungen wie beschrieben in US Provisional Application 60/984,023 bzw. US Provisional Application 61/061,273 und den jeweils darauf basierenden Patentanmeldungen, die bezüglich der darin offenbarten entsprechenden Verbindungen auf dem Wege der Verweisung Bestandteil dieser Anmeldung werden.

Besonders bevorzugt sind Kombinationen mit Homoeriodictyol und dessen Natrium-, Kalium-, Calcium-, Magnesium- oder Zinksalzen, Divanillinen, Phloretin und/oder Hesperitin.

Ein weiterer Aspekt der vorliegenden Erfindung betrifft ein Verfahren zum synergistischen Verstärken des süßen Geschmacks eines süß schmeckenden Stoffes (A) oder eines sowohl süß als auch bitter schmeckenden Stoffes C, umfassend die Schritte, umfassend die Schritte Vermischen
i) des süß schmeckenden Stoffes (A) oder des sowohl süßen als auch bitter schmeckenden Stoffes (C) und
ii) - einer Verbindung der Formel (I),
   oder
   - eines Salzes einer Verbindung der Formel (I)
      oder
   - einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
      wobei
   - R¹ und R² bedeuten unabhängig voneinander Wasserstoff, Methyl oder Ethyl,
   - R³ und R⁴ bedeuten unabhängig voneinander Wasserstoff, Hydroxy, Methoxy oder Ethoxy und
   - die Konfiguration am chiralen Kohlenstoffatom ist (R) oder (S),
wobei i) und ii) in einem Gemisch in einem Verhältnis zueinander vorliegt, so dass der süße Geschmack des süß schmeckenden Stoffes (A) oder des sowohl süßen als auch bitter schmeckenden Stoffes (C) synergistisch verstärkt wird.

Bevorzugt ist ein Verfahren (wie oben beschrieben)
(a) zum synergistischen Verstärken des süßen Geschmacks
   und
(b) zum Maskieren oder Vermindern des bitteren Geschmackseindrucks
   eines sowohl süß als auch bitter schmeckenden Stoffes (C), umfassend die Schritte
   i) Bereitstellen eines sowohl süß als auch bitter schmeckenden Stoffes (C),
   ii) Bereitstellen
      - einer Verbindung der Formel (I),
         oder
      - eines Salzes einer Verbindung der Formel (I)
         oder
      - einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
         wie jeweils oben beschrieben,
         und
   iii) Vermischen der in Schritt i) und ii) bereitgestellten Komponenten sowie gegebenenfalls weiterer Komponenten zu einem Gemisch in einem Verhältnis zueinander, so dass (a) der süße Geschmack des sowohl süß als auch bitter schmeckenden Stoffes (C) synergistisch verstärkt wird und (b) der bittere Geschmackseindruck des sowohl süß als auch bitter schmeckenden Stoffes (C) maskiert oder vermindert wird.

Für weitere bevorzugte Ausgestaltungen eines erfindungsgemäßen Verfahrens, insbesondere hinsichtlich der bevorzugten Auswahl der Stoffe (A), (B) bzw. (C) sowie der Verbindungen der Formel (I) bzw. deren Salzen und Mischungen davon gilt das vorangehend Gesagte entsprechend.

Nachfolgend wird die Erfindung anhand von Beispielen weiter erläutert. Die Beispiele dienen zur Verdeutlichung der Erfindung, ohne den Schutzbereich der Patentansprüche dabei einzuschränken. Sofern nicht anders angegeben, beziehen sich sämtliche Angaben auf das Gewicht.

### Beispiele

### Allgemeine Arbeitsvorschrift AAV 1: Reduktion von Flavanonen zu den korrespondierenden Flavanen

Das Flavanon wird in Pyridin (1,0 ml/mmol Flavanon) vorgelegt und mit Hexamethyldisilazan (1,0 ml/mmol Flavanon) versetzt. Anschließend wird Trimethylchlorsilan (0,6 ml/mmol Flavanon) zugetropft und die Mischung für 30 Minuten bei Raumtemperatur gerührt. Die flüchtigen Bestandteile werden unter vermindertem Druck abdestilliert und der Rückstand in Toluol aufgenommen (10,0 ml/mmol Flavanon). Nach dem Abfiltrieren der unlöslichen Bestandteile, wird das Toluol unter vermindertem Druck wieder abdestilliert und der Rückstand in THF (2,5 ml/mmol Flavanon) aufgenommen. Die Reaktionslösung wird mit Lithiumborhydrid (0,5 mmol/mmol Flavanon) versetzt. Nach einer weiteren Stunde Rühren bei Raumtemperatur werden 0,5 mg Methylorange sowie Natriumcyanoborhydrid (1mmol/mmol Flavanon) hinzugefügt. Anschließend wird 1N Salzsäure langsam unter Gasentwicklung bis zum Farbumschlag (rot) titriert und die Mischung wird durch weitere langsame Zugabe der Salzsäure in diesem pH-Bereich gehalten. Nach etwa zwei Stunden und einer Zugabe von etwa 1,0 bis 1,5 ml 1N Salzsäure pro mmol Flavanon verbleibt die rote Färbung dauerhaft und die Reaktion wird für weitere 12 Stunden bei Raumtemperatur gerührt. Das THF wird unter vermindertem Druck wieder abdestilliert und der Rückstand mit Ethylacetat extrahiert. Die organische Phase wird mit 1N Salzsäure, destilliertem Wasser sowie gesättigter Natriumchloridlösung gewaschen, über Natriumsulfat getrocknet und eingeengt. Das Produkt wird durch Kristallisation (Essigester/Hexan) und/oder Säulenchromatographie (Essigester/Pentan) aufgereinigt.

### Beispiel 1: 3',7-Dihydroxy-4'-methoxyflavan (Verbindung 2)

### a) 1-[2-Hydroxy-4-(tetrahydro-pyran-2-yloxy)-phenyl]-3-[4-methoxy-3-(tetrahydro-pyran-2-yloxy)-phenyl]-propenon

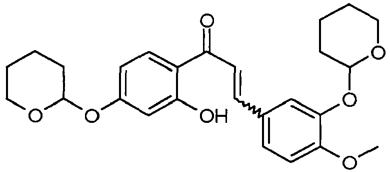

Eine Lösung aus 25,65 g (109,6 mmol) 1-[2-Hydroxy-4-(tetrahydro-pyran-2-yloxy)-phenyl]-ethanon und 25,56 g (108,2 mmol) 4-Methoxy-3-(tetrahydro-pyran-2-yloxy)-benzaldehyd in 250 ml Methanol wird mit 8,25 g (122,9 mmol) einer 85%igen Kalilauge versetzt und für 16 Stunden unter Rückfluss erhitzt. Nach Abkühlen auf Raumtemperatur wird die Lösung mit 9,0 g (149,9 mmol) Eisessig versetzt und das Reaktionsgemisch am Rotationsverdampfer eingeengt.

Der Rückstand wird in 300 ml Essigester und 200 ml Wasser aufgenommen, die organische Phase abgetrennt und erneut mit 200 ml Wasser gewaschen, über Natriumsulfat getrocknet und zur Trockene eingeengt. Das Aldolkondensationsprodukt wurde ohne weitere Aufreinigung in der nächsten Stufe eingesetzt (siehe b)).

### b) 7-Hydroxy-2-(3-hydroxy-4-methoxy-phenyl)-chroman-4-one

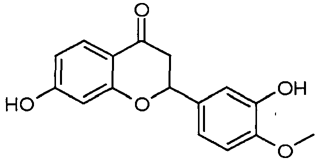

54,0 g des oben genannten (siehe a)) Aldolkondensationsproduktes wurden in 300 ml Methanol aufgenommen und mit 10 ml 37%iger Salzsäure versetzt. Nachdem die Reaktionsmischung für weitere 14 Stunden bei Raumtemperatur gerührt wurde, wird der Reaktionsansatz mit Wasser auf ein Volumen von 1,5 Liter verdünnt, wobei das korrespondierende, entschützte Chalkon als brauner Feststoff ausfällt. Dieser Feststoff wird in Essigester aufgenommen, über Natriumsulfat getrocknet und erneut bis zur Trockene eingeengt.

Der Rückstand wird in 250 ml Methanol gelöst und langsam unter Eiskühlung mit 50 ml Trifluormethansulfonsäure versetzt. Nach weiteren drei Stunden Rühren bei Raumtemperatur wird das Reaktionsgemisch am Rotationsverdampfer eingeengt und anschließend in 600 ml Essigester aufgenommen. Die organische Phase wird zweimal mit gesättigter Kochsalzlösung gewaschen, über Natriumsulfat getrocknet und am Rotationsverdampfer eingeengt. Das Rohprodukt wog 19,1 g und enthielt laut LC-MS ein Gemisch aus dem erwarteten cyclischen Chromanon und der gewünschten offenkettigen Verbindung, welche ohne Aufreinigung weiter umgesetzt wird (siehe c)).

### c) 3',7-Dihydroxy-4'-methoxyflavan (Verbindung 2)

19,1 g des oben genannten Chromanon/Chalkon Gemischs werden in 200 ml THF gelöst und mit 12,57 (200,0 mmol) Natriumcyanoborhydrid versetzt. Dann wurde die Reaktionsmischung durch langsame Zugabe von 1N Salzsäure langsam unter Gasentwicklung auf einen pH-Bereich von 2,8 bis 3,2 eingestellt, welcher durch weitere Zugabe von 1N Salzsäure eingehalten wurde. Verändert sich der pH-Wert der Reaktion nicht mehr, wird für weitere 40 Stunden bei Raumtemperatur nachgerührt. Das THF wird unter vermindertem Druck wieder abdestilliert und der Rückstand mit Ethylacetat extrahiert. Die organische Phase wird mit 1N Salzsäure, destilliertem Wasser sowie gesättigter Natriumchloridlösung gewaschen, über Natriumsulfat getrocknet und eingeengt. Das Rohprodukt wird erneut in Essigester aufgenommen, über 90 g Silica filtriert und anschließend eingeengt. Durch Umkristallisation (Essigester/Hexan 1:2) erhält man schließlich 2,20 g (8,1 mmol) des gewünschten Flavan-Derivats (Verbindung 2). Die Verbindung 2 lag dabei als racemische Mischung vor. Dasselbe gilt vorzugsweise für die in den Beispielen 3 bis 8 hergestellten erfindungsgemäß zu verwendenden Verbindungen der Formel (I).

**¹H-NMR (400 MHz, CDCl₃):** 2,03 (m, 1H); 2,15 (m, 1H); 2,71 (m, 1H); 2,88 (m, 1H); 3,89 (s, 3H); 4,67 (s, 1H); 4,95 (dd, *J* = 2,6 Hz, *J* = 10,1 Hz, 1H); 5,63 (s, 1H); 6,38 (dd, *J* = 2,6 Hz, *J* = 9,0 Hz, 1H); 6,38 (d, *J* = 2,3 Hz, 1H); 6,85 (d, *J* = 8,3, 1H); 6,89 - 6,94 (kB, 2H); 6,99 (d, *J* = 2,1 Hz, 1H) ppm.
**¹³C-NMR (100 MHz, DMSO)**: 23,5 (CH₂); 29,3 (CH₂); 55,6 (CH₃); 76,5 (CH); 102,6 (CH); 107,8 (CH); 111,9 (CH); 112,1 (C); 113,3 (CH); 116,7 (CH); 129,7 (CH); 132,2 (C); 146,3 (C); 147,0 (C); 155,3 (C); 156,4 (C) ppm.
**Massenspektrum (EI):** m/z (%) = 273 (17); 272 (M^{•+}, 100); 163 (14); 162 (31); 150 (87); 137 (38); 135 (48); 123 (14); 107 (17); 77 (14).

### Beispiel 2: (2S)-3',7-Dihydroxy-4'-methoxyflavan (Verbindung S-2)

Die Verbindung wurde analog zu Masaoud et al., (1995) "Flavonoids of dragon's blood from Dracaena cinnabari", Phytochemistry, Band 38, 745-749 aus "Dragons blood" isoliert. Der Drehwert der Probe betrug [·]_{D}²⁴ = -45 ° (MeOH, c = 0,30). Laut chiraler GC lag die Probe zu >90 % als (2S)-Enantiomer vor (Säule DAcTBS-β-CD, Trägergas 2 mL

H₂/min, Temperatur-Programm: 100-4-220°C, RT 54 min, Vergleich gegen die racemische Probe aus Beispiel 1). Die MS- und NMR-Daten sind identisch zur Probe aus Beispiel 1.

### Beispiel 3: 4',7-Dihydroxy-3'-methoxyflavan (Verbindung 3)

### a) (E)-1-(4-Benzyloxy-3-methoxy-phenyl)-3-(2,4-bis-benzyloxy-phenyl)-propenon

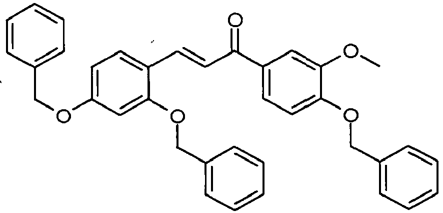

Eine Lösung aus 6,7 g (21,0 mmol) 2,4-Bis-benzyloxybenzaldehyd und 5,4 g (21,1 mmol) 1-(4-Benzyloxy-3-methoxy-phenyl)ethanon in 125 ml Ethanol werden mit 0,69 g (10,5 mmol) Ätzkali versetzt und für 15 Stunden unter Rückfluss erhitzt, wobei das Produkt bereits ausfällt. Nach Ende der Reaktion werden 50 ml Wasser sowie 300 ml Ethanol zugegeben und das Gemisch erneut unter Rückfluss erhitzt, bis der gesamte Feststoff wieder gelöst ist. Nach Abkühlen wird der nun wieder ausgefallene Feststoff abfiltriert, mit genügend Wasser gewaschen und anschließend bei 70 °C getrocknet. Das Rohprodukt wiegt 10,5 g und wird ohne weitere Aufreinigung in der nächsten Stufe eingesetzt (siehe b)).

### b) 4-[3-Hydroxy-3-(4-hydroxy-3-methoxy-phenyl)-propyl]-benzene-1,3-diol

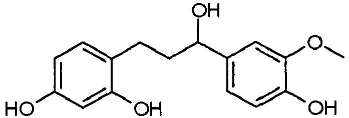

10,5 g des oben genannten Rohprodukts (siehe a)), d.h. des Aldolkondensationsprodukts, werden in 150 ml trockenem THF aufgenommen und unter Stickstoffatmosphäre auf 10 °C abgekühlt. Dann werden 0,36 g (9,5 mmol) Lithiumaluminiumhydrid zügig zugegeben, wobei die Innentemperatur, d.h. die Temperatur im Reaktionskolben, 20 °C nicht überschreiten soll. Anschließend wird für fünf Stunden bei Raumtemperatur gerührt, bevor das Reaktionsgemisch auf 0 °C abgekühlt und nacheinander mit 0,4 ml Wasser, 0,4 ml 15%iger Natriumhydroxydlösung sowie 1,2 ml Wasser versetzt wird. Anschließend werden 20,0 g Natriumsulfat zugegeben und die Mischung für weitere 30 Minuten gerührt. Schließlich werden die Feststoffe abfiltriert und die Lösung am Rotationsverdampfer eingeengt.
Das Zwischenprodukt wird in einem Lösungsmittelgemisch aus 100 ml Ethanol und 100 ml Essigsäureethylester aufgenommen und nach Zugabe von 3,0 g Hydrierkatalysator (5% Palladium auf Kohle) für 9 Stunden bei Raumtemperatur unter Wasserstoffatmosphäre gerührt, wobei 950 ml Wasserstoff verbraucht werden. Der Katalysator wird durch Filtration über Kieselgur abgetrennt und man erhält nach Entfernen des Lösungsmittels 3,94 g der gewünschten entschützten, offenkettigen Verbindung, welche ohne Aufreinigung in die anschließende Zyklisierung eingesetzt wird (siehe c)).

### c) 4',7-Dihydroxy-3'-methoxyflavan (Verbindung 3)

3,94 g der oben beschriebenen entschützten, offenkettigen Verbindung (siehe b)) werden in 100 ml THF gelöst und mit 5,0 ml 85%iger Phosphorsäure für vier Stunden unter Rückfluss erhitzt. Dann wird die Reaktionsmischung auf Raumtemperatur abgekühlt und unter Rühren mit 350 ml Essigsäureethylester sowie 200 ml Wasser versetzt. Die organische Phase wird abgetrennt und mit 100 ml gesättigter Kochsalzlösung gewaschen. Nach Entfernen des Lösungsmittels und anschließender Säulenchromatographie an Silica (Essigsäureethylester/Hexan 1:1) werden 1,36 g (5,0 mmol) des gewünschten 4',7-Dihydroxy-3'-methoxyflavan (Verbindung 3) als farbloser bis leicht rosa Feststoff erhalten.

**¹H-NMR (400 MHz, CDCl₃):** 2,05 (m, 1H); 2,15 (m, 1H); 2,73 (m, 1H); 2,91 (m, 1H); 3,90 (s, 3H); 4,71 (s, 1H); 4,95 (dd, *J* = 2,6 Hz, *J* = 10,3 Hz, 1H); 5,62 (m, 1H); 6,37 - 6,41 (kB, 2H); 6,88 - 6,95 (kB, 4H) ppm.
**¹³C-NMR (100 MHz, CDCl₃):** 24,6 (CH₂); 30,1 (CH₂); 55,9 (CH₃); 78,0 (CH); 103,5 (CH); 107,9 (CH); 108,7 (CH); 114,2 (C); 114,3 (CH); 119,2 (CH); 130,2 (CH); 133,6 (C); 145,4 (C); 146,6 (C); 154,8 (C); 156,0 (C) ppm.
**Massenspektrum (EI):** m/z (%) = 273 (14); 272 (M^{•+}, 61); 151 (12); 150 (100); 137 (34); 135 (32); 123 (17); 122 (16); 107 (13); 28 (38).

### Beispiel 4: 3',4',5-Trihydroxy-7-methoxyflavan (Verbindung 13)

Nach **AAV 1** wurden 4,00 g (13,3 mmol) Sterubin zu 0,28 g (1,0 mmol) des korrespondierenden Flavans bzw. Flavan-Derivats (Verbindung 13) umgesetzt.

**¹H-NMR (400 MHz, DMSO):** 1,83 (m, 1H); 2,02 (m, 1H); 2,54 (m, 2H); 3,62 (s, 3H); 4,79 (dd, *J* = 2,1 Hz, *J* = 10,0 Hz, 1H); 5,86 (d, *J* = 2,5 Hz, 1H); 5,98 (d, *J* = 2,5 Hz, 1H); 6,64 (dd, *J* = 2,1 Hz, *J* = 6,7 Hz, 1H); 6,71 (d, *J* = 8,1, 1H); 6,78 (d, *J* = 2,0, 1H); 8,6 - 9,4 (kB, 3H) ppm.
**¹³C-NMR (100 MHz, DMSO):** 18,9 (CH₂); 28,8 (CH₂); 54,7 (CH₃); 76,6 (CH); 92,4 (CH); 93,7 (CH); 101,8 (C); 113,6 (CH); 115,1 (CH); 117,1 (CH); 132,4 (C); 144,7 (C); 144,9 (C); 156,1 (C); 156,3 (C); 158,4 (C) ppm.
**Massenspektrum (EI):** m/z (%) = 288 (M^{•+}, 28); 192 (9); 165 (21); 153 (100); 152 (7); 148 (19); 140 (10); 137 (9); 136 (11); 65 (8).

### Beispiel 5: 4',5,7-Trihydroxyflavan (Verbindung 14)

Nach **AAV 1** wurden 2,36 g (8,7 mmol) Naringinin zu 1,02 g (3,9 mmol) des korrespondierenden Flavans bzw. Flavan-Derivats (Verbindung 14) umgesetzt.

**¹H-NMR (400 MHz, CD₃OD):** 1,91 (m, 1H); 2,08 (m, 1H); 2,58 (m, 1H); 2,68 (m, 1H); 4,81 (dd, *J* = 2,0 Hz, *J* = 10,3 Hz, 1H); 5,85 (d, *J* = 2,3 Hz, 1H); 5,93 (d, *J* = 2,3 Hz, 1H); 6,78 (m, 2H); 7,21 (m, 2H); 8,6 - 9,4 (kB, 3H) ppm.
**¹³C-NMR (100 MHz, CD₃OD):** 20,5 (CH₂); 30,8 (CH₂); 78,9 (CH); 95,98 (CH); 96,05 (CH); 102,6 (C); 116,1 (CH); 128,5 (CH); 134,3 (C); 157,3 (C); 157,4 (C); 158,00 (C); 158,03 (C) ppm.
**Massenspektrum (EI):** m/z (%) = 269 (18); 258 (M^{•+}, 77); 152 (16); 139 (94); 133 (29); 120 (100); 107 (28); 91 (29); 55 (16); 28 (33).

### Beispiel 6: 3',5,7-Trihydroxy-4'-methoxyflavan (Verbindung 15)

Nach **AAV 1** wurden 3,02 g (10,0 mmol) Hesperitin zu 1,74 g (6,0 mmol) des korrespondierenden Flavans bzw. Flavan-Derivats (Verbindung 15) umgesetzt.

**¹H-NMR (400 MHz, DMSO):** 1,81 (m, 1H); 2,02 (m, 1H); 2,46 - 2,54 (kB, 2H); 3,76 (s, 3H); 4,81 (dd, *J* = 2,1 Hz, *J* = 9,4 Hz, 1H); 5,70 (d, *J* = 2,3 Hz, 1H); 5,89 (d, *J* = 2,3 Hz, 1H); 6,76 (ddd, *J* = 0,6 Hz, *J* = 2,1 Hz, *J* = 6,8 Hz, 1H); 6,81 (d, *J* = 2,1, 1H); 6,89 (d, *J* = 8,4, 1H); 8,91 (s, 1H); 8,97 (s, 1H); 9,16 (s, 1H) ppm.
**¹³C-NMR (100 MHz, DMSO):** 18,7 (CH₂); 28,9 (CH₂); 55,6 (CH₃); 76,2 (CH); 94,2 (CH); 94,9 (CH); 100,1 (C); 111,9 (CH); 113,3 (CH); 116,8 (CH); 134,4 (C); 146,2 (C); 147,0 (C); 155,9 (C); 156,0 (C); 156,2 (C) ppm.
**Massenspektrum** (LC-MS: *C18; 100x2,1 mm; 0,2 ml*/*min; H₂O*/*CH₃CN 100*/*0 → 0*/*100*): m/z (%) = 334 (15); 333 (100); 331 (8); 289 (5); 288 (34).

### Beispiel 7: 4',5,7-Trihydroxy-3'-methoxyflavan (Verbindung 16)

Nach **AAV 1** wurden 3,02 g (10,0 mmol) Homoeriodyctiol zu 0,70 g (2,4 mmol) des korrespondierenden Flavans bzw. Flavan-Derivats (Verbindung 16) umgesetzt.

**¹H-NMR (400 MHz, DMSO):** 1,87 (m, 1H); 2,03 (m, 1H); 2,43 - 2,59 (kB, 2H); 3,77 (s, 3H); 4,80 (dd, *J* = 2,0 Hz, *J* = 10,2 Hz, 1H); 5,69 (d, *J* = 2,3 Hz, 1H); 5,88 (d, *J* = 2,3 Hz, 1H); 6,75 (d, *J* = 6,8 Hz, 1H); 6,79 (dd, *J* = 1,9 Hz, *J* = 6,8 Hz, 1H); 6,94 (d, *J* = 1,9, 1H); 8,90 (bs, 1H); 8,93 (bs, 1H); 9,15 (s, 1H) ppm.
**¹³C-NMR (100 MHz, DMSO):** 19,1 (CH₂); 28,9 (CH₂); 55,5 (CH₃); 76,6 (CH); 94,2 (CH); 94,9 (CH); 100,1 (C); 110,4 (CH); 115,0 (CH); 118,6 (CH); 132,6 (C); 145,9 (C); 147,3 (C); 156,0 (C); 156,16 (C); 156,18 (C) ppm.
**Massenspektrum** (LC-MS: *C18; 100x2,1 mm; 0,2 ml*/*min; H₂O*/*CH₃CN 100*/*0 → 0*/*100*): m/z (%) = 342 (3); 334 (7); 330 (3); 289 (6); 288 (100).

### Beispiel 8: 4',7-Dihydroxyflavan (Verbindung 1)

### a) (E)-1-(4-Benzyloxy-phenyl)-3-(2,4-bis-benzyloxyphenyl)-propenone

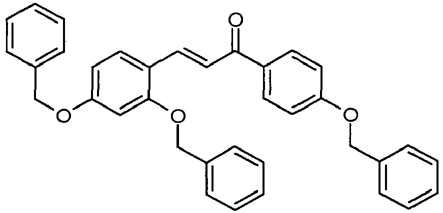

Eine Lösung aus 31,8 g (0,1 mol) 2,4-Bis-benzyloxybenzaldehyd und 22,6 g (0,1 mol) 1-(4-Benzyloxy-phenyl)ethanon in 300 ml Ethanol werden mit 6,6 g (0,1 mol; 85%ig in H₂O Ätzkali versetzt und für 8 Stunden unter Rückfluss erhitzt, wobei das Produkt bereits ausfällt. Nach Ende der Reaktion wird der Feststoff abfiltriert, der Filterrückstand viermal mit je 75 ml einer 1:1 Mischung aus Wasser und Ethanol gewaschen und anschließend bei 70 °C getrocknet. Das Rohprodukt wiegt 48,8 g und wird ohne weitere Aufreinigung in der nächsten Stufe eingesetzt (siehe b)).

### b) 4-[3-Hydroxy-3-(4-hydroxy-phenyl)-propyl]-benzene-1,3-diol

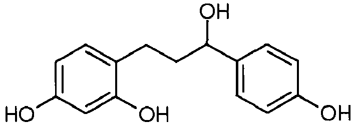

21,0 g des oben genannten Rohprodukts (siehe a)), d.h. des Aldolkondesationsprodukts, werden in 100 ml trockenem THF sowie 300 ml trockenem Diethylether aufgenommen und unter Stickstoffatmosphäre auf 10 °C abgekühlt. Dann werden 0,76 g (20,0 mmol) Lithiumaluminiumhydrid zügig zugegeben, wobei die Innentemperatur, d.h. die Temperatur im Reaktionskolben, 20 °C nicht überschreiten soll. Anschließend wird für fünf Stunden bei Raumtemperatur gerührt, bevor das Reaktionsgemisch wiederum auf 10 °C abgekühlt und mit 10%-iger Salzsäure sowie Eiswasser versetzt wird. Nach Abtrennen der organischen Phase wird diese mit Wasser und gesättigter Natriumhydrogencarbonatlösung gewaschen, über Natriumsulfat getrocknet und am Rotationsverdampfer eingeengt. Das Zwischenprodukt wird in einem Lösungsmittelgemisch aus 300 ml Isopropanol und 100 ml Tetrahydrofuran aufgenommen und nach Zugabe von 5,0 g Hydrierkatalysator (5% Palladium auf Kohle) für 8 Stunden bei 35°C unter Wasserstoffatmosphäre gerührt, wobei 2950 ml Wasserstoff verbraucht werden. Der Katalysator wird durch Filtration über Kieselgur abgetrennt und man erhält nach Entfernen des Lösungsmittels 20,4 g der gewünschten entschützten, offenkettigen Verbindung, welche ohne Aufreinigung in die anschließende Zyklisierung eingesetzt wird (siehe c)).

### c) 4',7-Dihydroxyflavan (Verbindung 1)

20,4 g der oben beschriebenen entschützten, offenkettigen Verbindung (siehe b)) werden in 400 ml THF gelöst und mit 10,0 ml 85%iger Phosphorsäure für sechs Stunden unter Rückfluss erhitzt. Dann wird die Reaktionsmischung auf Raumtemperatur abgekühlt und mit 500 ml Essigsäureethylester sowie 1000 ml Wasser versetzt. Die organische Phase wird abgetrennt und zweimal mit je 300 ml Wasser gewaschen und über Natriumsulfat getrocknet. Nach Entfernen des Lösungsmittels und anschließender Säulenchromatographie an Silica (Essigsäureethylester/Hexan 1:1) werden 2,57 g (10,6 mmol) des gewünschten 4',7-Dihydroxyflavan (Verbindung 1) als farbloser bis leicht rosa Feststoff erhalten.

**¹H-NMR (400 MHz, DMSO):** 1,92 (m, 1H); 2,03 (m, 1H); 2,59 (m, 1H); 2,80 (m, 1H); 4,90 (dd, *J* = 2,2 Hz, *J* = 10,0 Hz, 1H); 6,17 (d, *J* = 2,4 Hz, 1H); 6,27 (dd, *J* = 2,5 Hz, *J* = 8,2 Hz, 1H); 6,75 (m, 2H); 6.86 (d, *J* = 2,5 Hz, 1H); 7,20 (m, 2H); 9.14 (bs, 1H); 9,38 (bs, 1H) ppm.
**¹³C-NMR (100 MHz, DMSO):** 23,8 (CH₂); 29,2 (CH₂); 76,7 (CH); 102,6 (CH); 107,8 (CH); 112,1 (C); 114,9 (CH); 127,3 (CH); 129,7 (CH); 131,8 (C); 155,4 (C); 156,3 (C); 156,8 (C) ppm.
**Massenspektrum** (LC-MS: EclipseC18, *0,2 ml*/*min; H₂**O*/*CH₃**CN 100*/*0 → 5*/*95,* Wasser enthält 0,01% Ameisensäure): m/z (%) = 242 (12); 241 (68); 239 (5); 163 (5); 135 (39); 122 (8); 121 (100); 119 (77). Anwendungsbeispiel 1: Bitter-Reduzierung einer Bitterstofflösung

Um die Reduzierung (d.h. die Maskierung oder Verminderung) des Bitter-Eindrucks in einer Probe zu quantifizieren, wurde die Bitterkeit einer 500 ppm Coffein enthaltenden Lösung von einer Expertengruppe jeweils mit einer Probe verglichen, die 500 ppm Coffein und zusätzlich 50 ppm bzw. 100 ppm einer (hinsichtlich der Fähigkeit zur Bitter-Reduzierung) zu beurteilenden Substanz enthielt (Einstufung: 1 [nicht bitter] bis 10 [extrem bitter]).

Für die Auswertung, d.h. die Berechnung der Reduktion (in %) des Bittereindrucks wurden jeweils die Durchschnittswerte der Einschätzungen der Expertengruppe für die Coffeinlösung und die zu vergleichende, Coffein und eine zu beurteilende Substanz enthaltende Probe verwendet. 2,4-Dihydroxybenzoesäure (2,4-DHB) wurde dabei als Vergleich (Referenz) gemäß US 5,643,941 verwendet.

| **(Test-/Vergleichs-)Substanz** | **Coffein** (Bitterstoff) | **Bitter-Eindruck** | | **Reduktion des Bitter-Eindrucks** | **Bemerkung zur Sensorik der Probe** |
|---|---|---|---|---|---|
| | | **Coffein-Lösung** | **Probe** (Coffein + Substanz) | | |
| 100 ppm 2,4-DHB | 500 ppm | 5,1 ± 1,0 | 5,0 ± 1,0 | 3% | - |
| 50 ppm 7,4'-Dihydroxy-3'-methoxyflavan (Verbindung 2 aus Beispiel 1) | 500 ppm | 5,4 ± 1,5 | 3,9 ± 1,9 | 27 % (p < 0,05) | süß, alkoholisch, Lakritz |
| 50 ppm (2S)-7,4'-Dihydroxy-3'-methoxyflavan (Verbindung S-2 aus Beispiel 2) | 500 ppm | 4,7 ± 1,6 | 3,9 ± 1,5 | 16 % | - |
| 50 ppm 3',7-Dihydroxy-4'-methoxyflavan (Verbindung 3 aus Beispiel 3) | 500 ppm | 4,8 ± 2,3 | 4,1 ± 1,4 | 15 % | Leicht herb, süß |
| 50 ppm 4',5,7-Trihydroxyflavan (Verbindung 14 aus Beispiel | 500 ppm | 5,1 ± 2,1 | 3,8 ± 1,9 | 26 % (p < 0,05) | ethanolisch, fruchtig, grün |
| 50 ppm 3',5,7-Trihydroxy-4'-methoxyflavan (Verbindung 15 aus Beispiel 6) | 500 ppm | 4,4 ± 1,6 | 3,8 ± 1,5 | 15 % | - |
| 50 ppm 4',5,7-Trihydroxy-3'-methoxyflavan (Verbindung 16 aus Beispiel 7) | 500 ppm | 4,4 ± 1,6 | 4,1 ± 2,0 | 5 % | weicher, leicht alkoholisch |

### Anwendungsbeispiel 2: Verstärkung des Süß-Eindrucks einer Zuckerlösung

### Vergleichsversuch 1:

Um die Verstärkung des Süß-Eindrucks zu quantifizieren, wurde jeweils die Süße einer 5%igen Sucroselösung mit einer Probe, die 5 % Sucrose sowie 50 ppm bzw. 100 ppm einer erfindungsgemäß zu verwendenden Verbindung der Formel (I) enthielt, von einer Expertengruppe verglichen (Einstufung: 1 [nicht süß] bis 10 [extrem süß]).

Für die Auswertung, d.h. die Berechnung der Verstärkung (in %) des Süß-Eindrucks wurden jeweils die Durchschnittswerte der Einschätzungen der Expertengruppe für die Sucroselösung und die zu vergleichende, Sucrose und eine Verbindung der Formel (I) enthaltende Probe verwendet.

| **(Test-/Vergleichs-) Substanz** | **Sucrose** (Süßstoff) | **Süß-Eindruck (1-10)** | | **Verstärkung des Süß-Eindrucks** |
|---|---|---|---|---|
| | | **Sucrose-Lösung** | **Probe** (Sucrose + Substanz) | |
| 50 ppm Verbindung 2 (Beispiel 1) | 5% | 5,1 ± 0,9 | 8,1 ± 1,3 | 57 % (p < 0,001) |
| 50 ppm Verbindung S-2 (Beispiel 2) | 5% | 5,7 ± 1,2 | 7,8 ± 1,7 | 36 % (p < 0,001) |
| 50 ppm Verbindung 15 (Beispiel 6) | 5% | 4,8 ± 1,1 | 5,8 ± 1,6 | 21 % (p < 0,005) |
| 100 ppm Verbindung 1 (Beispiel 8) | 5% | 5,1 ± 1,4 | 6,2 ± 1,5 | 21 % (p < 0,05) |

### Vergleichsversuch 2:

Zur Beurteilung der synergistisch süß verstärkenden Wirkung einer erfindungsgemäß zu verwendenden Verbindung der Formel (I) wurde folgender Vergleichsversuch durchgeführt: Die Eigensüße der Verbindung 2 aus Beispiel 1, die in verschiedenen Konzentrationen in reiner Form in Wasser aufgelöst wurde (0,0025 bzw. 0,0050 Gew.-% in Wasser), wurde anhand einer Vergleichsreihe verschiedener Sucrosekonzentrationen in Wasser (0; 0,25; 0,5; 0,75; 1; 1,5; 2; 3; 4 und 5 Gew.-% Sucrose in Wasser) von einer Expertengruppe (Panelisten) bestimmt.

Die Panelisten waren aufgefordert, jede Lösung der Verbindung 2 in Wasser gegen die Sucrose-Reihe zu testen und entsprechend ihrem Süßegrad einzuordnen (Sucroseäquivalenz). Aus den über die Panelisten gemittelten Ergebnissen konnte die Eigensüße der Verbindung 2 für die oben erwähnten Konzentrationen bestimmt werden. Neben der Sucroseäquivalenz in Wasser (d.h. ohne Sucrose) wurde zudem die Sucroseäquivalenz in einer 5 %igen SucroseLösung bestimmt.

| **Konzentation der Verbindung 2 aus Beispiel 1 in Wasser [Gew.-%]** | **Bestimmung der Sucroseäquivalenz in Wasser (ohne Sucrose)** | **Sucroseäquivalenz in einer 5%igen Sucrose-Lösung** | |
|---|---|---|---|
| | | **Berechnung (5+x) %** | **Bestimmung** |
| 0,0025 | 0,84 % | 5,84 % | 6,8 % |
| 0,0050 | 1,45 % | 6,45 % | 8,2 % |

Bereits bei geringer, an sich nicht süßer Konzentration von ca. 25 ppm an Verbindung 2 (aus Beispiel 1) konnte eine deutliche synergistische Süßverstärkung festgestellt werden. Rein rechnerisch war zu erwarten, dass eine 5%ige Sucroselösung (d.h. eine Sucroselösung, die sensorisch definitionsgemäß 5 % Sucroseäquivalenten entspricht), die 25 ppm bzw. 50 ppm an Verbindung 2 (aus Beispiel 1) enthält, durch einen rein additiven Effekt der Eigensüße der Verbindung 2 (siehe die bestimmte Sucroseäquivalenz in Wasser) eine Süße von ca. 5,84 bzw. 6,45 Sucroseäquivalenten ergibt. Die sensorische Bewertung einer solchen 5%igen Sucroselösung mit 25 ppm bzw. 50 ppm an Verbindung 2 (aus Beispiel 1) durch die Panelisten ergab jedoch gemittelt eine Sucroseäquivalenz von 6,8 % bzw. 8,2 %. Dies entspricht in Anbetracht der rein rechnerisch zu erwartenden Werte einem Synergismus bzw. einem synergistischen Zusatzeffekt von ca. 17 % bzw. 27 %.

### Anwendungsbeispiel 3: Sprühgetrocknete Zubereitung als Halbfertigware zur Aromatisierung von Fertigwaren

| **Inhaltsstoff** | **Einsatz in Gew.-%** | | | | | | | | |
|---|---|---|---|---|---|---|---|---|---|
| **Zubereitung** | **A** | **B** | **C** | **D** | **E** | **F** | **G** | **H** | **I** |
| Trinkwasser | 60,8 | 60,8 | 56,4 | 60,8 | 60,8 | 60,8 | 60,8 | 60,8 | 60,8 |
| Maltodextrin aus Weizen | 24,3 | 24,3 | 24,3 | 24,3 | 24,3 | 24,3 | 24,3 | 24,3 | 24,3 |
| Gummi Arabicum | 6,1 | 6,1 | 6,1 | 6,1 | 6,1 | 6,1 | 6,1 | 6,1 | 6,1 |
| Verbindung 2 (Beispiel 1) | 8,8 | - | - | - | 6,6 | 5,5 | 3,3 | 4,4 | - |
| Verbindung S-2 (Beispiel 2) | - | 8,8 | - | - | - | - | - | - | - |
| Verbindung 14 (Beispiel 5) | - | - | 13,2 | - | - | - | - | - | - |
| Verbindung 15 (Beispiel 6) | - | - | - | 8,8 | - | - | - | - | - |
| Verbindung 1 (Beispiel 8) | - | - | - | - | - | - | - | - | 6,6 |
| Hesperetin | - | - | - | - | 2,2 | - | - | 1,1 | - |
| Homoeriodictyol-Natriumsalz | - | - | - | - | - | - | 5,5 | 3,3 | - |
| Phloretin | - | - | - | - | - | 3,3 | - | - | 2,2 |

Das Trinkwasser wird in einem Behälter vorgelegt und Maltodextrin und Gummi Arabicum werden darin gelöst. Anschließend werden die Aromastoffe mit einem Turrax in die Trägerstofflösung emulgiert. Die Temperatur der Sprühlösung sollte 30°C nicht überschreiten. Das Gemisch wird dann sprühgetrocknet (Solltemperatur Eingang: 185 bis 195 °C, Solltemperatur Ausgang: 70 bis 75 °C).

### Anwendungsbeispiel 4: Kombination mit Süßungsmitteln

90 g Sucrose und 10 g Tagatose werden mit 0,5 g einer sprühgetrockneten Halbfertigware aus Anwendungsbeispiel 3 (gemäß Zubereitung B) versetzt und vermischt. Das Produkt kann z.B. als Süßungsmittel mit Bitter-Maskierungseffekt für Kaffee oder Tee eingesetzt werden.

### Anwendungsbeispiel 5: Zuckerreduziertes Erfrischungsgetränk

- Zubereitung A:: Vergleichszubereitung mit 10 % Zucker
- Zubereitung B:: Vergleichszubereitung mit 8 % Zucker
- Zubereitung C:: Vergleichszubereitung mit 7 % Zucker
- Zubereitung D-G:: erfindungsgemäße gegenüber A (sowie für Zubereitungen E-G gegenüber B) zuckerreduzierte Zubereitungen mit 7 bzw. 8 % Zucker

| **Inhaltsstoff** | **Einsatz in Gew.-%** | | | | | | |
|---|---|---|---|---|---|---|---|
| **Zubereitung** | **A** | **B** | **C** | **D** | **E** | **F** | **G** |
| Zucker | 10 | 8 | 7 | 8 | 7 | 7 | 7 |
| Zitronensäure | 0.15 | 0.15 | 0.15 | 0.15 | 0.15 | 0.15 | 0.15 |
| Zitronenaroma | 0.1 | 0.1 | 0.1 | 0.1 | 0.1 | 0.1 | 0.1 |
| Verbindung 2, Beispiel 1 | - | - | - | 0.005 | 0.005 | 0.005 | 0.005 |
| Phloretin | - | - | - | - | - | 0.002 | - |
| Hesperetin | - | - | - | - | - | - | 0.001 |
| Wasser | ad 100 | | | | | | |

Die Zutaten wurden in der angegebenen Reihenfolge gemischt und mit Wasser auf 100 Gew.-% aufgefüllt. Die Mischungen werden in Glasflaschen gefüllt und carbonisiert.

Die Zubereitungen wurden anschließend sensorisch in verblindeten Duo-Tests geprüft und untereinander verglichen. Dabei wurde die Süße anhand einer Einstufung von 1 [nicht süß] bis 10 [extrem süß] von Experten eingeschätzt.

| **Vergleich (1. und 2. Probe)** | **Süß-Eindruck (1-10)** | | **Unterschied (Süß-Eindruck)** | **Signifikanz p** |
|---|---|---|---|---|
| | **1. Probe** | **2. Probe** | | |
| **Zubereitungen A und B** | 7,1 ± 1,4 | 4,6 ± 1,3 | -36 % | < 0,001 |
| **Zubereitungen A und D** | 6,3 ± 1,6 | 6,2 ± 1,9 | -1 % | > 0,95 |
| **Zubereitungen A und C** | 7,1 ± 1,8 | 4,5 ± 1,4 | -37 % | < 0,001 |
| **Zubereitungen A und E** | 7,1 ± 1,2 | 5,9 ± 1,6 | -16 % | < 0,05 |
| **Zubereitungen A und F** | 6,5 ± 1,6 | 6,0 ± 2,1 | -8 % | = 0,44 |
| **Zubereitungen A und G** | 6,5 ±1,3 | 6,4 ± 2,2 | -1 % | > 0,95 |

Demnach wurde durch Reduzieren der Zucker-Menge (um 2 bzw. 3 Gew.-% bei Zubereitung B bzw. C, bezogen auf das Gesamtgewicht der Zubereitung) eine Abnahme der Süße von ca. 36 bzw. 37 % (s. Tabelle) beobachtet.

Durch Hinzufügen einer geringen Menge einer erfindungsgemäß zu verwendenden Verbindung der Formel (I) allein (s. Zubereitungen D und E) oder in Kombination mit bekannten Aromastoffen zur Süßverstärkung (s. Zubereitungen F und G) konnte im Idealfall (A gegen D und A gegen G) aufgrund der synergistisch süß-verstärkenden Wirkung von Verbindung 2 kein relevanter Unterschied zwischen der Vollzucker-Zubereitung und der an Zucker ärmeren, erfindungsgemäßen Zubereitung festgestellt werden.

### Anwendungsbeispiel 6: Tee-Zubereitung

| **Inhaltsstoff** | **Einsatz in Gew.-%** | | |
|---|---|---|---|
| **Zubereitung** | **A** | **B** | **C** |
| schwarzer Tee, Ceylon, Blattware | 94,00 % | | |
| grüner Tee, China, Blattware | | 92,00 % | |
| Mate Tee, Peru, Blattware | | | 95,00 % |
| Halbfertigware A aus Anwendungsbeispiel 3 | 6 % | | |
| Halbfertigware G aus Anwendungsbeispiel 3 | | 8 % | |
| Halbfertigware H aus Anwendungsbeispiel 3 | | | 5 % |

Der Tee und die Halbfertigware werden gemischt und in Teebeutel aus Filterpapier abgepackt. Zur Anwendung wird ein Teebeutel in 100 - 250 ml kochendes Wasser aufgegossen und 2 - 5 Minuten ziehen gelassen.

### Anwendungsbeispiel 7: Verwendung in einem Soja-Getränk

Die Verbindung 2, Beispiel 1, bzw. Verbindung S-2, Beispiel 2, wurde in Ethanol vorgelöst und zu einer Sojamilch aus einem lokalen Supermarkt hinzugefügt. Die Mischung wurde zusammen mit einem Milcharoma im Becherglas verrührt.

| **Inhaltsstoff** | **Einsatz in Gew.-%** | | | |
|---|---|---|---|---|
| **Zubereitung** | **A** | **B** | **C** | **D** |
| Sojamilch (lokaler Supermarkt, nicht aromatisiert, unge- | 96,7 | 99,68 | 98,29 | 97,60 |
| Vanillearoma | 0,1 | 0,1 | | 0,05 |
| Milcharoma | | | 0,1 | 0,05 |
| Saccharose | 3 | | 1,5 | 2 |
| Rebaudiosid A 95 % | | 0,02 | 0,01 | |
| Emulgum | 0,1 | 0,1 | | 0,1 |
| 10 % Verbindung 2, Beispiel 1 in Ethanol | 0,1 | | 0,1 | |
| 10 % Verbindung S-2, Beispiel 2 in Ethanol | | 0,1 | | 0,1 |
| Hesperetin, 5 % in Ethanol | | | | 0,1 |

### Anwendungsbeispiel 8: Verwendung in einem Kaugummi

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Rebaudiosid A 98 % | 0,2 |
| | Emulgum^{®} (Colloides Naturels, Inc.) | 0,30 |
| C | Sorbitol, 70% | 14,00 |
| | Glycerin | 1,00 |
| D | Aroma, enthaltend 1 Gew.-% Verbindung 2, Beispiel 1, bezogen auf das Gesamtgewicht des Aromas | 1,00 |

Teile A bis D werden gemischt und intensiv geknetet. Die Rohmasse kann z.B. in Form von dünnen Streifen zu verzehrfertigen Kaugummis verarbeitet werden.

### Anwendungsbeispiel 9: Verwendung in einer Zahnpasta

| **Teil** | **Inhaltsstoff** | **Einsatz in Gew.-%** |
|---|---|---|
| A | demineralisiertes Wasser | 22,00 |
| | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Rebaudiosid A, 98 % | 0,10 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisertes Wasser | 4,53 |
| | Natriumlaurylsulfat | 1,50 |
| D | Aroma, enthaltend 1 Gew.-% Verbindung 2, Beispiel 1, bezogen auf das Gesamtgewicht des Aromas | 1,00 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C 30 min gut verrührt. Nach Entspannung ist die Zahnpasta fertig und kann abgefüllt werden.

### Anwendungsbeispiel 10: Zuckerfreie Hartkaramelle

| **Inhaltsstoff** | **Gehalt (%)** | | | |
|---|---|---|---|---|
| | **A** | **B** | **C** | **D** |
| Palatinit, Typ M | 75,00 | 74,00 | 75,50 | 75,00 |
| Zitronensäure | - | 1,0 | 0,5 | - |
| Wasser | 24,88 | 24,842 | 23,88 | 24,844 |
| Farbstoff gelb | - | 0,01 | - | - |
| Farbstoff rot | - | - | 0,01 | - |
| Farbstoff blau | 0,01 | - | - | 0,01 |
| Pfefferminzaroma | 0,1 | - | - | 0,1 |
| Zitronenaroma | - | 0,1 | - | - |
| Rotfruchtaroma | - | - | 0,1 | - |
| Rebaudiosid A 98 % | - | 0,040 | - | 0,040 |
| 3',7-Dihydroxy-4'-methoxyflavan (Verbindung 2, Beispiel 1) | 0,010 | 0,005 | 0,010 | 0,005 |
| Hesperetin | - | 0,001 | - | 0,001 |
| Phloretin | - | 0,002 | - | - |

Palatinit wurde mit Wasser gemischt und die Mischung bei 165 °C aufgeschmolzen und anschließend auf 115°C abgekühlt. Die übrigen Bestandteile wurden zugegeben und nach dem Durchmischen in Formen gegossen, nach dem Erstarren aus den Formen entfernt und anschließend einzeln verpackt.

### Anwendungsbeispiel 11: Zuckerreduzierter Kochpudding

- A:: Vergleichszubereitung mit 7,8 % Sucrosegehalt
- B:: Vergleichszubereitung mit (gegenüber A) reduziertem Sucrosegehalt
- C:: Erfindungsgemäße Zubereitung mit (gegenüber A) reduziertem Sucrosegehalt und 3',7-Dihydroxy-4'-methoxyflavan (Verbindung 2, Beispiel 1)
- D:: Erfindungsgemäße Zubereitung mit (gegenüber A) reduziertem Sucrosegehalt, D-Tagatose und 3',7-Dihydroxy-4'-methoxyflavan (Verbindung 2, Beispiel 1)

| **Inhaltsstoff** | **Zubereitung (Angaben in Gew.-%)** | | | |
|---|---|---|---|---|
| | **A** | **B** | **C** | **D** |
| **Sucrose** | **7,8 %** | **5,4 %** | **5,4 %** | **5,4 %** |
| Stärke | 3,0 % | 3,0 % | 3,0 % | 3,0 % |
| Magermilch-pulver | 1,5 % | 1,5 % | 1,5 % | 1,5 % |
| Aubygel MR50 | 0,5 % | 0,5 % | 0,5 % | 0,5 % |
| Vanilleschoten-Extrakt, sprühgetrocknet, Symrise | 0,1 % | 0,1 % | 0,1 % | 0,1 % |
| Verbindung 2, Beispiel 1 | - | - | 0,01 % | 0,005 % |
| D-Tagatose | - | - | - | 0,1 % |
| Milch 1,5 % Fettanteil | ad 100 % | ad 100 % | ad 100 % | ad 100 % |

Die festen Stoffe wurden vorgelegt und mit der Milch aufgerührt. Die Mischung wurde auf 95 °C für 2 min unter gutem Rühren aufgewärmt, abgefüllt und auf 5 - 8 °C abgekühlt.

Bei Zubereitung C konnte bei Verkostung durch Versuchspersonen die Süße der 7,8 % Sucrose enthaltenden Vergleichszubereitung A (bei einem etwas verzögerten Süßeeindruck) erreicht werden. Zubereitung C war im Vergleich zur Vergleichszubereitung B wesentlich stärker süß. Zubereitung D war vergleichbar mit C, zeigte aber eine verbesserte Anfangssüße.

### Anwendungsbeispiel 12: Fettarme Joghurts

- A:: Vergleichszubereitung mit 10 % Sucrose
- B, C:: Erfindungsgemäße Zubereitungen mit Süßstoffmischung und 3',7-Dihydroxy-4'-methoxyflavan (Verbindung 2, Beispiel 1)

| **Inhaltsstoff** | **Zubereitung (Angaben als Gew.-%)** | | |
|---|---|---|---|
| | **A** | **B** | **C** |
| Sucrose | 10% | 8% | 6% |
| Tagatose | - | - | 0,5% |
| Fructose | - | - | 0,5% |
| (Verbindung 2, Beispiel 1 | - | 0,05 % | 0,025 % |
| Hesperetin | - | - | 0,005 % |
| Joghurt, 0,1 % Fett | ad 100 % | ad 100 % | ad 100 % |

Die Inhaltsstoffe wurden gemischt und bei 5°C gekühlt.

### Anwendungsbeispiel 13: Verwendung zusammen mit Süßstoffen in fettarmen Joghurts

- A:: Vergleichszubereitung mit Süßstoffmischung
- B-D:: Erfindungsgemäße Zubereitungen mit Süßstoffmischung und 3',7-Dihydroxy-4'-methoxyflavan (Verbindung 2, Beispiel 1)

| **Inhaltsstoff** | **Zubereitung (Angaben in Gew.-%)** | | | |
|---|---|---|---|---|
| | **A** | **B** | **C** | **D** |
| D-Tagatose | 0,482 % | 0,482 % | 0,482 % | - |
| Sucralose | 0,003 % | 0,003 % | 0,003 % | - |
| Aspartam | 0,005 % | 0,005 % | 0,005 % | - |
| Acesulfam K | 0,01 % | 0,01 % | 0,01 % | - |
| Rebaudiosid A 98% | - | - | - | 0,050 % |
| Verbindung 2, Beispiel 1 | - | 0,05 % | 0,025 % | 0,025 % |
| Hesperetin | - | - | 0,015 % | 0,015 % |
| Phloretin | - | - | 0,005 % | 0,005 % |
| Joghurt, 0,1 % Fett | ad 100 % | ad 100 % | ad 100 % | ad 100 % |

Die Inhaltsstoffe wurden gemischt und bei 5°C gekühlt.

### Anwendungsbeispiel 14: Milchmischgetränke

- A, B:: Vergleichszubereitungen mit Zucker
- C, D:: Erfindungsgemäße Zubereitungen mit Zucker und 3',7-Dihydroxy-4'-methoxyflavan (Verbindung 2, Beispiel 1)

| **Inhaltsstoff** | **Zubereitung (Angaben in Gew.-%)** | | | |
|---|---|---|---|---|
| | **A** | **B** | **C** | **D** |
| Sucrose | 10,0 | 8,0 | 8,0 | 7,0 |
| Fructose | - | - | - | 0,5 |
| Verbindung 2, Beispiel 1 | - | - | 0,05 | 0,025 |
| Hesperetin | - | - | - | 0,025 % |
| Phloretin | - | - | - | 0,005 % |
| H-Milch, 1,5 % Fett | ad 100 % | | | |

Die Inhaltsstoffe wurden gemischt, mit Milch aufgefüllt, gut gerührt, in Flaschen abgefüllt und bei 5°C gekühlt gelagert.

### Anwendungsbeispiel 15: Zuckerreduziertes Tomatenketchup

- A:: Vergleichszubereitung mit Zucker
- B:: Vergleichszubereitung mit (gegenüber A) reduziertem Zuckeranteil
- C-H:: Erfindungsgemäße Zubereitungen mit (gegenüber A) reduziertem Zuckeranteil und 3',7-Dihydroxy-4'-methoxyflavan (Verbindung 2, Beispiel 1)

| | **Zubereitung (Angaben in Gew.-%)** | | | | | | | |
|---|---|---|---|---|---|---|---|---|
| **Inhaltsstoff** | **A** | **B** | **C** | **D** | **E** | **F** | **G** | **H** |
| Kochsalz | 2 | 2 | 2 | 2 | 2 | 2 | 2 | 2 |
| Stärke, Farinex WM 55 | 1 | 1 | 1 | 1 | 1 | 1 | 1 | 1 |
| Sucrose | 12 | 9,6 | 9,2 | 8,4 | 9,6 | 9,6 | 8,4 | 8,4 |
| Tomaten-Konzentrat 2-fach | 40 | 40 | 40 | 40 | 30 | 30 | 30 | 30 |
| Glucosesirup 80 Brix | 18 | 18 | 18 | 18 | 18 | 18 | 18 | 18 |
| Branntweinessig 10% | 7 | 7 | 7 | 7 | 3 | 3 | 3 | 3 |
| Verbindung 2, Beispiel 1, 2,5%ig in 1,2-Propylenglycol | | | 0,2 | 0,1 | 0,1 | 0,2 | 0,1 | 0,1 |
| Hesperetin 2,5%ig in 1,2-Propylenglycol | | | | | 0,1 | | 0,2 | |
| Phloretin 2,5% in 1,2-Propylenglycol | | | | 0,2 | 0,2 | | | 0,2 |
| Wasser | zu 100 % auffüllen | | | | | | | |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 16: Zuckerreduzierte Eiscremes

- A:: Vergleichszubereitung mit Zucker
- B:: Vergleichszubereitung mit (gegenüber A) reduziertem Zuckeranteil
- C-F:: Erfindungsgemäße Zubereitungen mit (gegenüber A) reduziertem Zuckeranteil und Hesperetin

| **Inhaltsstoff** | **Zubereitung (Gehalt in Gew.-%)** | | | | | |
|---|---|---|---|---|---|---|
| | **A** | **B** | **C** | **D** | **E** | **F** |
| Pflanzenfett, Schmelzbereich 35 - 40 °C | 20,00 | 20,00 | 20,00 | 20,00 | 20,00 | 20,00 |
| Zucker (Saccharose) | 12,00 | 8,00 | 8,00 | 8,00 | 8,00 | 8,00 |
| Magermilchpulver | 5,00 | 5,00 | 5,00 | 5,00 | 5,00 | 5,00 |
| Glucosesirup 72 % Trockensubstanz | 5,00 | 5,00 | 5,00 | 5,00 | 5,00 | 5,00 |
| Emulgator SE 30 (Grindstedt Products, Dänemark) | 0,65 | 0,65 | 0,65 | 0,65 | 0,65 | 0,65 |
| Aroma, enthaltend 0,1 % Diacetyl und 1 % Vanillin | 0,20 | 0,20 | 0,20 | 0,20 | 0,20 | 0,20 |
| Verbindung 2, Beispiel 1, 2,5%ig in 1,2-Propylenglycol | | | 0,20 | 0,10 | 0,20 | 0,10 |
| Hesperetin 2,5%ig in 1,2-Propylenglycol | | | | 0,10 | | 0,10 |
| Phloretin 2,5% in 1,2-Propylenglycol | | | | | 0,05 | 0,05 |
| Magermilch | zu 100 % auffüllen | | | | | |

Das Pflanzenfett wurde auf 58°C erwärmt. Magermilch und Glucosesirup wurden auf 55° erhitzt und Zucker, Magermilchpulver sowie Emulgator und Aroma hinzugefügt und die Mischung ins Pflanzenfett gegeben. Die Mischung wurde mit Hilfe eines Durchflusshochdruckhomogenisators homogenisieret (180 / 50 bar). Die erhaltene Masse wurde 1 min lang bei 78°C temperiert, anschließend auf 2 - 4 °C abgekühlt und 10 h zur Reifung bei dieser Temperatur inkubiert. Danach wurde die gereifte Masse abgefüllt und bei -18°C eingefroren gelagert.

### Anwendungsbeispiel 17: Für Diabetiker geeignetes Eis

Es wurde ein für Diabetiker geeignetes Eis aus folgenden Zutaten hergestellt und in Portionen zu je 95 mL in Becher abgefüllt:
Eingedickte entrahmte Milch, Fructosesirup, Erdbeerstücke und Erbeerpüree (15%), Pflanzenfett, Diätschokoladensplitter (3,5%, mit Emulgator Sojalecithin), Molkenerzeugnis, Rote Bete Saft, Johannisbrotkernmehl, Guarkernmehl, Carrageen, Emulgator (E 471), Gelatine, Säuerungsmittel Citronensäure, Erdbeer-Aroma (enthaltend 1 Gew.-% 3',7-Dihydroxy-4'-methoxyflavan (Verbindung 2, Beispiel 1), bezogen auf das Gesamtgewicht des Erdbeer-Aromas), Farbstoff Carotin.

Nährwert (pro 95 mL):
Eiweiss 1,8 g, Kohlenhydrate 13,3 g (davon Fructose 9,5 g), Fett 4,2 g.

### Anwendungsbeispiel 18: Diätschokolade auf Basis von Maltit

Es wurde eine für Diabetiker geeignete Schokolade aus folgenden Zutaten hergestellt und in rechteckige Tafeln gegossen:
Maltit, Haselnussmasse, Kakaobutter, Magermilchpulver, Kakaomasse, Inulin, Butterreinfett, Emulgator Sojalecithine, Vanille-Aroma (enthaltend Vanilleschoten-Extrakt, Vanillin und 1 Gew.-% 3',7-Dihydroxy-4'-methoxyflavan (Verbindung 2, Beispiel 1), bezogen auf das Gesamtgewicht des Vanille-Aromas).

Nährwert (pro 100 g): Eiweiss 8 g, Kohlenhydrate 43 g (davon Maltit 34 g), Fett 34 g.

### Anwendungsbeispiel 19: Diätschokolade auf Basis von Fructose

Es wurde eine für Diabetiker geeignete Schokolade aus folgenden Zutaten hergestellt und in rechteckige Tafeln gegossen:
Kakaomasse, Fructose, Magermilchpulver, Kakaobutter, Inulin, Butterreinfett, Emulgator Sojalecithin, Walnüsse, Speisesalz, Vanille-Aroma (enthaltend Vanillin und 1 Gew.-% 3',7-Dihydroxy-4'-methoxyflavan (Verbindung 2, Beispiel 1), bezogen auf das Gesamtgewicht des Vanille-Aromas).

Nährwert (pro 100 g):
Eiweiss 8,8 g, Kohlenhydrate 34 g (davon Fructose 23 g, Lactose 7,5 g, Saccharose 1,4 g), Fett 36 g; Ballaststoffe 18,5 (davon 12,2 g Inulin); Natrium: 0,10 g. Kakao-Anteil mindestens 50 Gew.-%.

### Anwendungsbeispiel 20: Zuckerreduzierte Müslimischung

| **Nr.** | **Inhaltsstoff** | **A (Gew.-%), Vergleichszubereitung** | **B (Gew.-%), erfindungsgemäß** |
|---|---|---|---|
| 1 | Haferflocken | 17,00 | 18,90 |
| 2 | Knusprige Haferflocken Cluster | 10,00 | 12,00 |
| 3 | Reis Crispies | 16,90 | 17,80 |
| 4 | Cornflakes | 16,50 | 17,50 |
| 5 | Korinthen | 3,50 | 3,50 |
| 6 | Haselnüsse, zerhackt | 2,50 | 2,50 |
| 7 | Glucose Sirup aus Weizen, DE 30 | 9,50 | 9,50 |
| 8 | Saccharose | 20,00 | 14,00 |
| 9 | Wasser | 4,00 | 4,00 |
| 10 | Citronensäurepulver, wasserfrei | 0,10 | 0,10 |
| 11 | Aroma, enthaltend 2,5 Gew.-% an 3',7-Dihydroxy-4'-methoxyflavan (Verbindung 2, Beispiel 1), bezogen auf das Gesamtgewicht des Aromas | - | 0,20 |

Bestandteile Nr. 1 bis 6 werden jeweils in einer Drehtrommel gemischt (Mix 1). Bestandteile Nr. 7 bis 9 werden erwärmt und Bestandteil Nr. 10 (in Rezeptur B zudem Bestandteil Nr. 11) wird zugeben (Mix 2). Mix 2 wird zu Mix 1 gegeben und anschließend wird gut gemischt. Zuletzt wird die resultierende Müslimischung auf ein Backblech gegeben und in einem Ofen bei 130°C für 8 Minuten getrocknet.

### Anwendungsbeispiel 21: Zuckerreduzierte Fruchtgummis

| **Inhaltsstoff** | **A (Gew.-%), Vergleichszubereitung** | **(Gew.-%), erfindungsgemaß** |
|---|---|---|
| Wasser | 23,70 | 25,70 |
| Saccharose | 34,50 | 8,20 |
| Glucosesirup, DE 40 | 31,89 | 30,09 |
| Iso Syrup C* Tru Sweet 01750 (Cerestar GmbH) | 1,50 | 2,10 |
| Gelatine 240 Bloom | 8,20 | 9,40 |
| Polydextrose (Litesse^{®} Ultra, Danisco Cultor | - | 24,40 |
| Gelber und roter Farbstoff | 0,01 | 0,01 |
| Citronensäure | 0,20 | |
| Kirscharoma, enthaltend 2,5 Gew.-% an 3',7-Dihydroxy-4'-methoxyflavan (Verbindung 2, Beispiel 1) bezogen auf das Aroma | - | 0,10 |

Anmerkung: Polydextrose ist ein selbst nicht süß schmeckendes Polysaccharid mit niedrigem Brennwert.

### Anwendungsbeispiel 22: Schoko-Cappuccino-Eiscreme

| **Inhaltsstoff** | **A (Gex.-%), Vergleichszubereitung** | **B (Gew.-%) erfindunsgemäß** |
|---|---|---|
| Glucose-Fructose-Sirup | 14,10 | 14,10 |
| Saccharose | 10,00 | 7,50 |
| Magermilchpulver | 5,00 | 5,00 |
| Sahne (36% Fettanteil) | 24,00 | 24,00 |
| Emulgator und Stabilisator Cremodan^{®} 709VEG (Da- | 0,50 | 0,50 |
| Kakaopulver | 5,975 | 5,975 |
| Carrageenan | 0,025 | 0,025 |
| Wasser | 40,20 | 42,50 |
| Cappuccino-Aroma | 0,20 | 0,20 |
| 3',7-Dihydroxy-4'-methoxyflavan (Verbindung 2, Beispiel 1) 2,5%ig in 1,2-Propylenglycol / Ethanol | - | 0,20 |

### Anwendungsbeispiel 23: Gelatinekapseln zum Direktverzehr

| **Inhaltsstoff** | **A (Gew.-%)** | **B (Gew.-%)** | **C (Gew.-%)** |
|---|---|---|---|
| **Gelatinehülle:** | | | |
| Glycerin | 2,014 | 2,014 | 2,014 |
| Gelatine 240 Bloom | 7,91 | 7,91 | 7,91 |
| Sucralose | 0,065 | 0,065 | 0,065 |
| Allura Rot | 0,006 | 0,006 | 0,006 |
| Brillantblau | 0,005 | 0,005 | 0,005 |

| **Kernzusammensetzung:** | | | |
|---|---|---|---|
| Pflanzenöl-Triglycerid (Kokosöl - Fraktion) | 79,49 | 68,55 | 58,55 |
| Orangen-Aroma, enthaltend 1 Gew.-% Dihydroxy-4'-methoxyflavan (Verbindung 2, Beispiel 1), bezogen auf das Gesamtgewicht des Aromas | 10,0 | 20,0 | 28,65 |
| Rebaudiosid A 98 % | 0,05 | 0,05 | - |
| 2-Hydroxypropylmenthylcarbonat | 0,33 | 0,20 | - |
| 2-Hydroxyethylmenthylcarbonat | - | 0,20 | 1,00 |
| (1R,3R,4S) Menthyl-3-carbonsäure-N-ethylamid (WS-3) | - | 0,55 | 0,50 |
| (-)-Menthonglycerinacetal (Frescolat MGA) | - | 0,30 | 0,80 |
| Vanillin | 0,07 | - | 0,10 |

Die zum Direktverzehr geeigneten Gelatinekapseln wurden gemäß WO 2004/050069 hergestellt und hatten einen Durchmesser von 5 mm, das Gewichtsverhältnis von Kernmaterial zu Hüllmaterial lag bei 90 : 10. Die Kapseln öffneten sich im Mund innerhalb von weniger als 10 Sekunden und lösten sich vollständig innerhalb von weniger als 50 Sekunden auf.

## Patentansprüche

1. Verwendung
- einer Verbindung der Formel (I), 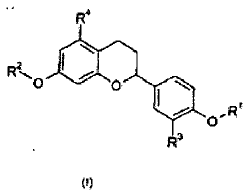 oder
- eines Salzes einer Verbindung der Formel (I)
oder
- einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
oder
- eines pflanzlichen Extrakts umfassend eine besagte Verbindung der Formel (I), ein besagtes Salz einer Verbindung der Formel (I) oder eine besagte Mischung,
wobei für die Gruppen R¹, R², R³ und R⁴ sowie die Konfiguration in der Verbindung der Formel (I) bzw. unabhängig voneinander in jeder Verbindung der Formel (I) gilt:
- R¹ und R² bedeuten unabhängig voneinander Wasserstoff, Methyl oder Ethyl,
- R³ und R⁴ bedeuten unabhängig voneinander Wasserstoff, Hydroxy, Methoxy oder Ethoxy und
- die Konfiguration am chiralen Kohlenstoffatom ist (R) oder (S), zur synergistischen Verstärkung des süßen Geschmacks eines süß schmeckenden Stoffes.

2. Verwendung nach Anspruch 1, wobei die bzw. eine, mehrere oder sämtliche Verbindung(en) der Formel (I) jeweils ausgewählt ist bzw. sind aus der Gruppe bestehend aus den folgenden Verbindungen 1 bis 16:
4',7-Dihydroxyflavan (Verbindung 1),
3',7-Dihydroxy-4'-methoxyflavan (Verbindung 2),
4',7-Dihydroxy-3'-methoxyflavan (Verbindung 3),
4'-Hydroxy-7-methoxyflavan (Verbindung 4),
4',7-Dimethoxy-3'-hydroxyflavan (Verbindung 5),
3',7-Dimethoxy-4'-hydroxyflavan (Verbindung 6),
3',4'-Dihydroxy-7-methoxyflavan (Verbindung 7),
5,7-Dimethoxy-4'-hydroxyflavan (Verbindung 8),
4'-Hydroxy-3',5,7-trimethoxyflavan (Verbindung 9),
3',4'-Dihydroxy-5,7-dimethoxyflavan (Verbindung 10),
4',5-Dihydroxy-3',7-dimethoxyflavan (Verbindung 11),
3',4',5,7-Tetrahydroxyflavan (Verbindung 12),
3',4',5-Trihydroxy-7-methoxyflavan (Verbindung 13),
4',5,7-Trihydroxyflavan (Verbindung 14),
3',5,7-Trihydroxy-4'-methoxyflavan (Verbindung 15),
4',5,7-Trihydroxy-3'-methoxyflavan (Verbindung 16), wobei unabhängig voneinander in jeder Verbindung 1 bis 16 die Konfiguration am chiralen Kohlenstoffatom (R) oder (S) ist.

3. Verwendung nach Anspruch 1, wobei eine Mischung aus zwei unterschiedlichen Verbindungen der Formel (I)verwendet wird, die ausgewählt sind aus der Gruppe bestehend aus den Verbindungen aus Anspruch 2,
wobei
- die Gruppen R¹ bis R⁴ der beiden Verbindungen identisch sind und
- die Konfiguration am chiralen Kohlenstoffatom der einen Verbindung (R) ist und die Konfiguration am chiralen Kohlenstoffatom der anderen Verbindung (S) ist.

4. Verwendung nach einem der Ansprüche 1 oder 2, wobei die bzw. eine Verbindung der Formel (I) (S)-3',7-Dihydroxy-4'-methoxyflavan (Verbindung S-2) ist.

5. Verwendung eines Salzes oder einer Mischung aus
- zwei oder mehr unterschiedlichen Salzen von Verbindungen der Formel (I),
oder
- einer oder mehreren unterschiedlichen Verbindungen der Formel (I),
und einem oder mehreren unterschiedlichen Salzen von Verbindungen der Formel (I),
nach Anspruch 1,
wobei das bzw. die Gegenkation(en) des bzw. eines, mehrerer oder sämtlicher der Salze von Verbindungen der Formel (I) ausgewählt ist bzw. sind aus der Gruppe bestehend aus Na⁺, K⁺, NH₄⁺, Ca²⁺, Mg²⁺, Al³⁺ und Zn²⁺.

6. Verwendung nach einem der vorangehenden Ansprüche, in einer Mischung umfassend
- einen süß schmeckenden Stoff (A) und einen unangenehm schmeckenden Stoff (B)
oder
- einen sowohl süß als auch unangenehm schmeckenden Stoff (C),
(a) zur synergistischen Verstärkung des süßen Geschmacks des süß schmeckenden Stoffes (A) bzw. (C)und
(b) zum Maskieren oder Vermindern des unangenehmen Geschmackseindrucks des unangenehm schmeckenden Stoffes (B) bzw. (C),
wobei der unangenehme Geschmackseindruck des unangenehm schmeckenden Stoffes (B) bzw.
(c) ein bitterer Geschmackseindruck ist.

7. Verwendung nach Anspruch 6, wobei
- der bitter schmeckende Stoff (B) oder
- der sowohl süß als auch bitter schmeckende Stoff (C) ausgewählt ist aus der Gruppe bestehend aus Steviolglycosiden.

8. Verwendung nach einem der vorangehenden Ansprüche in einer zur oralen Aufnahme bestimmten kosmetischen oder einer der Ernährung, der Mundpflege oder dem Genuss dienenden Zubereitung.

9. Aromakomposition zur synergistischen Verstärkung des süßen Geschmacks eines süß schmeckenden Stoffes (A) und zum Maskieren oder Vermindern eines bitteren Geschmackseindrucks eines bitter schmeckenden Stoffes (B), vorzugsweise zur synergistischen Verstärkung des süßen Geschmacks und zum Maskieren oder Vermindern des bitteren Geschmackseindrucks eines sowohl süß als auch bitter schmeckenden Stoffes (C), umfassend oder bestehend aus
(i) - einer Verbindung der Formel (I)
oder
- einem Salz einer Verbindung der Formel (I)
oder
- einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
oder
- einem pflanzlichen Extrakt umfassend eine besagte Verbindung der Formal (I), ein besagtes Salz einer Verbindung der Formel (I) oder eine besagte Mischung, wie jeweils in einem der Ansprüche 1 bis 5 beschrieben, sowie
(ii) einem oder mehreren Aroma- und/oder Geschmacksstoffen, ausgewählt aus der Gruppe bestehend aus Vanillin, Ethylvanillin, Ethylvanillinisobutyrat, 2,5-Dimethyl-4-hydroxy-3(2H)-furanon und Derivate, Homofuronol, Maltol und Derivate, Cumarin und Derivate, gamma-Lactone, delta-Lactone, Methylsorbat, Divanillin, 4-Hydroxy-2-ethyl-5-methyl-3(2H)furanon, 4-Hydroxy- 5-ethyl-2-methyl-3(2H)furanon, 2-Hydroxy-3-methyl-2-cyclopentenone, 3-Hydroxy-4,5-dimethyl-2(5H)-furanon, Fruchtester und Fruchtlactone, 4-(p-Hydroxyphenyl)-2-butanon, 1,1-Dimethoxy-2,2,5-trimethyl-4-hexan, 2,6-Dimethyl-5-hepten-1-al und Phenylacetaldehyd,
und/oder
(iii) einer oder mehreren
- Substanz(en) zum Maskieren oder Vermindern eines bitteren Geschmackeindrucks, ausgewählt aus der Gruppe bestehend aus Natriumsalze, Homoeriodictyol oder dessen Natriumsalze, 2,4-Dihydroxybenzoesaeurevanillylamid, gamma-Aminobuttersäure, Pellitorin und Gingerdione und/oder
- Substanz(en) zum Verstärken eines süßen Geschmackeindrucks, ausgewählt aus der Gruppe bestehend aus Hesperetin, Hydroxyphenylalkadionen, Deoxybenzoinen, 4-Hydroxychalkonen, Propenylphenylglycoside und Divanillinen.

10. Zubereitung, umfassend die Bestandteile
(I) - eine Verbindung der Formel (I), oder
- ein Salz einer Verbindung der Formel (I)
oder
- eine Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
wie jeweils in einem der Ansprüche 1 bis 5 beschrieben,
und
(II) einen oder mehrere süß schmeckende Stoffe (A) und/oder sowohl süß als auch bitter schmeckende Stoffe (C), wobei der bzw. die Stoffe (A) bzw. (C) keine Verbindung der Formel (I) oder ein Salz davon ist bzw. sind,
und wobei die Gesamtmenge an Verbindungen der Formel (I) bzw. Salzen der Verbindung der Formel (I) in der Zubereitung ausreicht, den süßen Geschmackseindruck des bzw. der Stoffe (A) bzw. (C) synergistisch zu verstärken.

11. Zubereitung nach Anspruch 10, wobei die Zubereitung eine der Ernährung, der Mundpflege oder dem Genuss dienende oder kosmetische oder zur oralen Aufnahme bestimmte pharmazeutische Zubereitung ist, wobei die Zubereitung bezogen auf das Gesamtgewicht der Zubereitung vorzugsweise 0,0001 Gew.-% (1 ppm) bis 0,5 Gew.-% (5000 ppm), bevorzugt 0,0001 Gew.-% (1 ppm) bis 0,1 Gew.-% (1000 ppm), besonders bevorzugt 0,001 Gew.-% (10 ppm) bis 0,05 Gew.-% (500 ppm), an Bestandteil (I) umfasst.

12. Zubereitung nach Anspruch 10, wobei die Zubereitung eine zur Herstellung einer der Mundpflege oder dem Genuss dienenden oder einer kosmetischen Zubereitung oder einer zur oralen Aufnahme bestimmten pharmazeutischen Zubereitung geeignete Halbfertigware ist, wobei die Halbfertigware vorzugsweise bezogen auf das Gesamtgewicht der Halbfertigware 0,0001 Gew.-% bis 95 Gew.-%, bevorzugt 0,001 bis 80 Gew.-%, besonders bevorzugt 0,01 Gew.-% bis 50 Gew.-%, an Bestandteil (I) umfasst.

13. Zubereitung nach einem der Ansprüche 10 bis 12, wobei Bestandteil (II)
- einen oder mehrere sowohl süß als auch bitter schmeckende Stoffe (C) und/oder
- einen oder mehrere bitter schmeckende Stoffe (B), wobei der bzw. die Stoffe (B) keine Verbindung der Formel (I) oder ein Salz davon ist bzw. sind, umfasst,
und wobei die Gesamtmenge an Verbindungen der Formel (I) bzw. Salzen der Verbindung der Formel (I) in der Zubereitung ausreicht, den bitteren Geschmackseindruck des bzw. der Stoffe (B) bzw. (C) zu maskieren oder vermindern,
wobei vorzugsweise einer, mehrere oder vorzugsweise sämtliche der sowohl süß als auch bitter schmeckenden Stoffe (C) ausgewählt sind aus der Gruppe bestehend aus Steviolglycosiden.

14. Zubereitung nach einem der Ansprüche 10 bis 13, wobei
- Bestandteil (I) in Form eines pflanzlichen Extrakts und/oder
- Bestandteil (II) in Form eines pflanzlichen Extrakts aus Stevia ssp.

15. Verfahren zum
synergistischen Verstärken des süßen Geschmacks eines süß schmeckenden Stoffes (A) oder eines sowohl süß als auch bitter schmeckenden Stoffes (C)
umfassend die Schritte
Vermischen
ii) des süß schmeckenden Stoffes (A) oder des sowohl süßen als auch bitter schmeckenden Stoffes (C) und
ii) - einer Verbindung der Formel (I),
oder
- eines Salzes einer Verbindung der Formel (I)
oder
- einer Mischung aus zwei oder mehr unterschiedlichen Verbindungen der Formel (I), zwei oder mehr unterschiedlichen Salzen von Verbindungen der Formel (I) oder einer oder mehreren unterschiedlichen Verbindungen der Formel (I) und einem oder mehreren unterschiedlichen Salzen von Verbindungen der Formel (I),
wobei
- R¹ und R² bedeuten unabhängig voneinander Wasserstoff, Methyl oder Ethyl,
- R³ und R⁴ bedeuten unabhängig voneinander Wasserstoff, Hydroxy, Methoxy oder Ethoxy und
- die Konfiguration am chiralen Kohlenstoffatom ist (R) oder (S),
wobei i) und ii) in einem Gemisch in einem Verhältnis zueinander vorliegt, so dass der süße Geschmack des süß schmeckenden Stoffes (A) oder des sowohl süßen als auch bitter schmeckenden Stoffes (C) synergistisch verstärkt wird.

## Claims

1. Use of
(i) compound of formula (I) 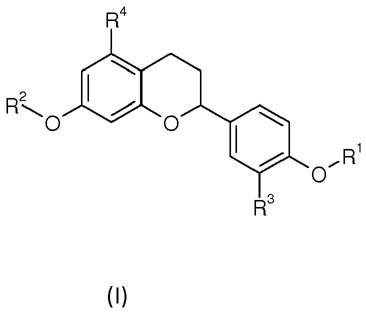 or
(ii) or a salt of a compound of formula (I)
or
(iii) a mixture of two or more different compounds of formula (I), two or more different salts of compounds of formula (I) or one or more different compounds of formula (I) and one or more different salts of compounds of formula (I),
or
(iv) a plant extract comprising said compound of formula (I), said salt of a compound of formula (I) or a said mixture,
wherein the following meanings apply to the groups R¹, R², R³ and R⁴ as well as to the configuration in the compound of formula (I) or independently of one another in each compound of formula (I):
(v) R¹ and R² independently of one another represent hydrogen, methyl or ethyl,
(vi) R³ and R⁴ independently of one another represent hydrogen, hydroxyl, methoxy or ethoxy, and
(vii) the configuration at the chiral carbon is (R) or (S), for synergistically enhancing the sweet taste of a sweet-tasting substance.

2. Use according to claim 1, wherein one, several or all of the compound (s) of formula (I) is in each case or are selected from the group consisting of the following compounds 1 to 16:
4',7-dihydroxyflavan (compound 1),
3',7-dihydroxy-4'-methoxyflavan (compound 2),
4',7-dihydroxy-3'-methoxyflavan (compound 3),
4'-hydroxy-7-methoxyflavan (compound 4),
4',7-dimethoxy-3'-hydroxyflavan (compound 5),
3',7-dimethoxy-4'-hydroxyflavan (compound 6),
3',4'-dihydroxy-7-methoxyflavan (compound 7),
5,7-dimethoxy-4'-hydroxyflavan (compound 8),
4'-hydroxy-3',5,7-trimethoxyflavan (compound 9),
3',4'-dihydroxy-5,7-dimethoxyflavan (compound 10),
4',5-dihydroxy-3',7-dimethoxyflavan (compound 11),
3',4',5,7-tetrahydroxyflavan (compound 12),
3',4',5-trihydroxy-7-methoxyflavan (compound 13),
4',5,7-trihydroxyflavan (compound 14),
3',5,7-trihydroxy-4'-methoxyflavan (compound 15),
4',5,7-trihydroxy-3'-methoxyflavan (compound 16),
wherein independently of one another in each compound is 1 to 16, the configuration at the chiral carbon atom is (R) or (S).

3. Use according to claim 1, wherein a mixture of two different compounds of formula (I) selected from the group consisting of the compounds according to claim 2 is used,
wherein
- R¹ to R⁴ of the two compounds are identical and
- the configuration at the chiral carbon atom of one compound is (R) and the configuration at the chiral carbon atom of the other compound is (S).

4. Use according to one of claims 1 or 2, wherein the compound(s) of formula (I) is/are (S)-3',7-dihydroxy-4'-methoxyflavan (compound S-2).

5. Use of a salt or a mixture of
- two or more different salts of compounds of formula (I),
or
- one or more different compounds of formula (I), and one or more different salts of compounds of formula (I) according to claim 1,
wherein the counter cation(s) of the or one, more or all the salts of compounds of formula (I) is/are selected from the group consisting of Na⁺, K⁺, NH₄⁺, Ca²⁺, Mg²⁺, Al³⁺ and Zn²⁺.

6. Use according to one of the preceding claims, in a mixture comprising
- a sweet-tasting substance (A) and an unpleasant-tasting substance (B)
or
- a both sweet and unpleasant-tasting substance (C),
(a) for synergistically enhancing a sweet taste of the sweet-tasting substance (A) or (C) and
(b) for masking or reducing an unpleasant taste sensation of the unpleasant-tasting substance (B) or (C).
wherein the unpleasant taste sensation of the unpleasant-tasting substance (B) or
(c) is a bitter taste sensation.

7. Use according to claim 6, wherein
- the bitter-tasting substance (B) or
- the both sweet and bitter-tasting substance (C) is selected from the group consisting of stevioglycoside.

8. Use according to one of the preceding claims in a preparation intended for oral ingestion, cosmetic or nutrition, oral care or enjoyment comprising the method according to claim 1.

9. Aroma composition for synergistically enhancing the sweet taste of a the sweet-tasting substance (A) and for masking or reducing the bitter taste sensation of the bitter-tasting substance (B), preferably for synergistically enhancing the sweet taste and for masking or reducing the bitter taste sensation of a both sweet and bitter-tasting substance (C), comprising or consisting of
(i) - compound of formula (I)
oder
- a salt of a compound of formula (I)
oder
- a mixture of two or more different compounds of formula (I), two or more different salts of compounds of formula (I) or one or more different compounds of formula (I) and one or more different salts of compounds of formula (I),
or
- a plant extract comprising said compound of formula (I), said salt of a compound of formula (I) or a said mixture according to one of claims 1 to 5, and
(ii) one or more flavoring and/or taste-imparting substances selected from the group consisting of vanillin, ethyl vanillin, ethylvanillinisobutyrate, 2,5-dimethyl-4-hydroxy-3(2H)-furanone and derivatives, homofuronol, maltol and derivatives, coumarin and derivatives, gamma-lactones, delta-lactones, methylsorbate, divanillin, 4-hydroxy-2-ethyl-5-methyl-3(2H)furanone, 4-hydroxy-5-ethyl-2-methyl-3(2H)furanone, 2-hydroxy-3-methyl-2-cyclopentenones, 3-hydroxy-4,5-dimethyl-2(5H)-furanone, fruit esters and fruit lactones 4-(p-hydroxyphenyl)-2-butanone, 1,1-dimethoxy-2,2,5-trimethyl-4-hexane, 2,6-dimethyl-5-hepten-1-al and phenylacetaldehyde,
and/or
(iii) one or more
- substance(s) for masking or reducing a bitter taste sensation selected from the group consisting of sodium salts, homoeriodictyol or the sodium salts thereof, 2,4-dihydroxybenozoic acid vanillylamide, gamma-amino butyric acid, pellitorine and gingerdiones and/or
- substance(s) for enhancing a sweet taste sensation, selected from the group consisting of hesperetin, hydroxyphenylalkadiones, deoxybenzoins, 4-hydroxychalcones, propenylphenylglycosides and divanillins.

10. A preparation comprising the ingredients
(I) - a compound of formula (I) or
- the salt of a com-pound of formula (I)
or
- the mixture of two or more different compounds of formula (I), two or more different salts of compounds of formula (I) or one or more different compounds of formula (I) and one or more different salts of compounds of formula (I), according to to one of claims 1 to 5
and
(II) one or more sweet-tasting substances (A) and/or both sweet and bitter-tasting substances (C), wherein the substances(s) (A) or (C) is/are not a compound of formula (I) or a salt thereof,
and the total amount of compounds of formula (I) or salts of the compound of formula (I) in the preparation is sufficient for synergistically enhancing a sweet taste sensation of the substance(s) (A) or (C).

11. Preparation according to claim 10, wherein the preparation is a preparation used for nutrition, oral care or enjoyment or a cosmetic preparation or a pharmaceutical preparation intended for oral ingestion, wherein the preparation comprises preferably 0.0001% by weight (1 ppm) to 0.5% by weight (5000 ppm), in particular 0.0001% by weight (1 ppm) to 0.1% by weight (1000 ppm), most particularly 0.001% by weight (10 ppm) to 0.05% (500 ppm) of ingredient (I), based on the total weight of the preparation.

12. Preparation according to claim 10, wherein the preparation is a suitable semi-finished product for manufacturing of an oral care or enjoyment or a cosmetic preparation or a particular for oral pharmaceutical preparation, wherein the semi-finished product comprises preferably 0.0001% by weight to 95% by weight of ingredient (I), in particular 0.001% to 80% by weight, most particularly 0.01% by weight to 50% by weight, based on the total weight of the semi-finished product.

13. Preparation according to one of claims 10 or 12, wherein ingredient (II) comprises
- one or more both sweet and bitter-tasting substances (C) and/or
- one or more bitter-tasting substances (B), wherein (B) is/are not a compound or salt of formula (I),
and the total amount of compounds of formula (I) or salts of the compound of formula (I) in the preparation is sufficient to mask or reduce a bitter taste sensation of the substance(s) (B) or (C)
wherein preferably one, several or preferably all of both sweet and bitter-tasting substances (C) are selected from the group consisting of steviolglycosides.

14. Preparation according to one of claims 10 to 13, wherein
- Component (I) in the form of a plant extract and / or
- Component (II), in the form of a plant extract from Stevia *ssp*.

15. A process for
synergistically enhancing a sweet taste of a sweet-tasting substance (A) or of a both sweet and bitter-tasting substance (C)
comprising the steps of
mixing
(i) of a sweet-tasting substance (A) or of a both sweet and bitter-tasting substance (C) and
(ii) - a compound of formula (I)
or
- a salt of a compound of formula (I)
or
- a mixture of two or more different compounds of formula (I), two or more different salts of compounds of formula (I) or one or more different compounds of formula (I) and one or more different salts of compounds of formula (I),
wherein
- R¹ and R² independently of one another represent hydrogen, methyl or ethyl,
- R³ and R⁴ independently of one another represent hydrogen, hydroxyl, methoxy or ethoxy, and
- the configuration at the chiral carbon is (R) or (S),
wherein i) and ii) are blended in a ratio to one another such that the sweet taste of substance (A) or the both sweet and bitter-tasting substance (C) is synergistically enhanced.

## Revendications

1. Utilisation
(i) - d'un composé de formule (I) 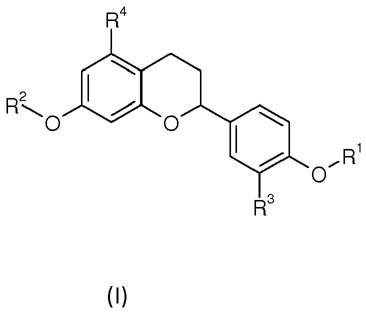 ou
- d'un sel d'un composé de formule (I)
ou
- d'un mélange de deux composés de formule (I) différents ou plus, de deux sels de composés de formule (I) différents ou plus, ou d'un ou de plusieurs composés de formule (I) différents et d'un ou de plusieurs sels de composés de formule (I) différents,
ou
- d'un extrait végétal comprenant un composé de formule (I) susmentionné, un sel d'un composé de formule (I) susmentionné ou un mélange susmentionné,
les indications suivantes étant applicables pour les groupes R¹, R², R³ et R⁴, ainsi que pour la configuration dans le composé de formule (I) ou indépendamment les uns des autres dans chaque composé de formule (I) :
- R¹ et R² signifient indépendamment l'un de l'autre hydrogène, méthyle ou éthyle,
- R³ et R⁴ signifient indépendamment l'un de l'autre hydrogène, hydroxy, méthoxy ou éthoxy, et
- la configuration de l'atome de carbone chiral est (R) ou (S), pour le renforcement synergique du goût sucré d'une substance à goût sucré.

2. Utilisation selon la revendication 1, dans laquelle le ou un, plusieurs ou tous les composés de formule (I) sont chacun choisis dans le groupe constitué par les composés 1 à 16 suivants :
le 4',7-dihydroxyflavane (composé 1),
le 3',7-dihydroxy-4'-méthoxyflavane (composé 2),
le 4',7-dihydroxy-3'-méthoxyflavane (composé 3),
le 4'-hydroxy-7-méthoxyflavane (composé 4),
le 4',7-diméthoxy-3'-hydroxyflavane (composé 5),
le 3',7-diméthoxy-4'-hydroxyflavane (composé 6),
le 3',4'-dihydroxy-7-méthoxyflavane (composé 7),
le 5,7-diméthoxy-4'-hydroxyflavane (composé 8),
le 4'-hydroxy-3',5,7-triméthoxyflavane (composé 9),
le 3',4'-dihydroxy-5,7-diméthoxyflavane (composé 10),
le 4',5-dihydroxy-3',7-diméthoxyflavane (composé 11),
le 3',4',5,7-tétrahydroxyflavane (composé 12),
le 3',4',5-trihydroxy-7-méthoxyflavane (composé 13),
le 4',5,7-trihydroxyflavane (composé 14),
le 3',5,7-trihydroxy-4'-méthoxyflavane (composé 15),
le 4',5,7-trihydroxy-3'-méthoxyflavane (composé 16), la configuration de l'atome de carbone chiral étant (R) ou (S) dans chaque composé 1 à 16 indépendamment les uns des autres.

3. Utilisation selon la revendication 1, dans laquelle un mélange de deux composés de formule (I) différents est utilisé, qui sont choisis dans le groupe constitué par les composés selon la revendication 2,
- les groupes R¹ à R⁴ des deux composés étant identiques et
- la configuration de l'atome de carbone chiral d'un composé étant (R) et la configuration de l'atome de carbone chiral de l'autre composé étant (S).

4. Utilisation selon l'une quelconque des revendications 1 ou 2, dans laquelle le ou un composé de formule (I) est le (S)-3',7-dihydroxy-4'-méthoxyflavane (composé S-2).

5. Utilisation d'un sel ou d'un mélange de
- deux sels de composés de formule (I) différents ou plus,
ou
- un ou plusieurs composés de formule (I) différents et un ou plusieurs sels de composés de formule (I) différents, selon la revendication 1,
le ou les contre-cations du ou d'un, de plusieurs ou de tous les sels de composés de formule (I) étant choisis dans le groupe constitué par Na⁺, K⁺, NH₄⁺, Ca²⁺, Mg²⁺, Al³⁺ et Zn²⁺

6. Utilisation selon l'une quelconque des revendications précédentes, dans un mélange comprenant :
- une substance à goût sucré (A) et une substance à goût désagréable (B),
ou
- une substance à goût sucré et également désagréable (C),
(a) pour le renforcement synergique du goût sucré de la substance à goût sucré (A) ou (C) et
(b) pour le masquage ou la réduction de l'effet gustatif désagréable de la substance à goût désagréable (B) ou (C),
l'effet gustatif désagréable de la substance à goût désagréable (B) ou (C) étant un effet gustatif amer.

7. Utilisation selon la revendication 6, dans laquelle
- la substance à goût amer (B) ou
- la substance à goût sucré et également amer (C) est choisie dans le groupe constitué par les glycosides de stéviol.

8. Utilisation selon l'une quelconque des revendications précédentes dans une préparation cosmétique destinée à l'administration orale ou dans une préparation pour l'alimentation, l'hygiène buccale ou le plaisir.

9. Composition aromatique pour le renforcement synergique du goût sucré d'une substance à goût sucé (A) et pour le masquage ou la réduction d'un effet gustatif amer d'une substance à goût amer (B), de préférence pour le renforcement synergique du goût sucré et pour le masquage ou la réduction de l'effet gustatif amer d'une substance à goût sucré et également amer (C), comprenant ou constituée par
(i) - un composé de formule (I)
ou
- un sel d'un composé de formule (I)
ou
- un mélange de deux composés de formule (I) différents ou plus, de deux sels de composés de formule (I) différents ou plus, ou d'un ou de plusieurs composés de formule (I) différents et d'un ou de plusieurs sels de composés de formule (I) diffé rents,
ou
- un extrait végétal comprenant un composé de formule (I) susmentionné, un sel d'un composé de formule (I) susmentionné ou un mélange susmentionné, à chaque fois tels que décrits dans l'une quelconque des revendications 1 à 5, et
(ii) une ou plusieurs substances aromatiques et/ou gustatives choisies dans le groupe constitué par la vanilline, l'éthylvanilline, l'isobutyrate d'éthylvanilline, la 2,5- di méthyl-4-hydroxy-3(2H)-furanone et ses dérivés, l'homofuronol, le maltol et ses dé rivés, la coumarine et ses dérivés, la gamma-lactone, la delta-lactone, le sorbate de méthyle, la divanilline, la 4-hydroxy-2-éthyl-5-méthyl-3(2H)furanone, la 4-hydroxy-5-éthyl-2-méthyl-3(2H)furanone, la 2-hydroxy-3-méthyl-2-cyclopenténone, la 3-hy droxy-4,5-diméthyl-2(5H)-furanone, les esters de fruits et les lactones de fruits, la 4-(p-hydroxyphényl)-2-butanone, le 1,1-diméthoxy-2,2,5-triméthyl-4-hexane, le 2,6-diméthyl-5-heptén-1-al et le phénylacétaldéhyde,
et/ou
(iii) une ou plusieurs
- substances pour le masquage ou la réduction d'un effet gustatif amer, choisies dans le groupe constitué par les sels de sodium, l'homoériodictyol ou ses sels de sodium, le vanillylamide de l'acide 2,4-dihydroxybenzo'ique, l'acide gamma-aminobutyrique, la pellitorine et la gingerdione, et/ou
- substances pour le renforcement d'un effet gustatif sucré, choisies dans le groupe constitué par l'hespérétine, les hydroxyphénylalcadiones, les désoxybenzo'ines, les 4-hydroxychalcones, les glycosides de propénylphényle et les divanillines.

10. Préparation, comprenant les constituants :
(I) - un composé de formule (I)
ou
- un sel d'un composé de formule (I)
ou
- un mélange de deux composés de formule (I) différents ou plus, de deux sels de composés de formule (I) différents ou plus, ou d'un ou de plusieurs composés de formule (I) différents et d'un ou de plusieurs sels de composés de formule (I) diffé rents,
à chaque fois tels que décrits dans l'une quelconque des revendications 1 à 5,
et
(II) une ou plusieurs substances à goût sucré (A) et/ou substances à goût sucré et éga lement amer (C), la ou les substances (A) ou (C) n'étant pas un composé de for mule (I) ou un sel de celui-ci,
et la totalité des composés de formule (I) ou des sels des composés de formule (I) dans la préparation suffisant pour renforcer synergiquement l'effet gustatif sucré de la ou des substances (A) ou (C).

11. Préparation selon la revendication 10, dans laquelle la préparation est une préparation pour l'alimentation, l'hygiène buccale ou le plaisir, ou une préparation cosmétique ou une préparation pharmaceutique destinée à une administration orale, la préparation comprenant par rapport au poids total de la préparation avantageusement 0,0001 % en poids (1 ppm) à 0,5 % en poids (5 000 ppm), de préférence 0,0001 % en poids (1 ppm) à 0,1 % en poids (1 000 ppm), de manière particulièrement préférée 0,001 % en poids (10 ppm) à 0,05 % en poids (500 ppm), de constituant (1).

12. Préparation selon la revendication 10, dans laquelle la préparation est un produit semi-fini approprié pour la fabrication d'une préparation pour l'hygiène buccale ou le plaisir, ou d'une préparation cosmétique, ou d'une préparation pharmaceutique destinée à une administration orale, le produit semi-fini comprenant par rapport au poids total du produit semi-fini avantageusement 0,0001 % en poids à 95 % en poids, de préférence 0,001 à 80 % en poids, de manière particulièrement préférée 0,01 % en poids à 50 % en poids, de constituant (1).

13. Préparation selon l'une quelconque des revendications 10 à 12, dans laquelle le constituant (II) comprend
- une ou plusieurs substances à goût sucré et également amer (C), et/ou
- une ou plusieurs substances à goût amer (B), la ou les substances (B) n'étant pas un composé de formule (I) ou un sel de celui-ci,
et la quantité totale des composés de formule (I) ou des sels des composés de formule (I) dans la préparation suffisant pour masquer ou réduire l'effet gustatif amer de la ou des substances (B) ou (C),
une, plusieurs ou de préférence toutes les substances à goût sucré et également amer (C) étant de préférence choisies dans le groupe constitué par les glycosides de stéviol.

14. Préparation selon l'une quelconque des revendications 10 à 13, dans laquelle
- le constituant (I) se présente sous la forme d'un extrait végétale et/ou
- le constituant (II) se présente sous la forme d'un extrait végétal de Stevia ssp.

15. Procédé de
renforcement synergique du goût sucré d'une substance à goût sucré (A) ou d'une substance à goût sucré et également amer (C),
comprenant les étapes suivantes :
le mélange
i) de la substance à goût sucré (A) ou de la substance à goût sucré et également amer (C), et
ii) - d'un composé de formule (I),
ou
- d'un sel d'un composé de formule (I),
ou
- d'un mélange de deux composés de formule (I) différents ou plus, de deux sels de composés de formule (I) différents ou plus, ou d'un ou de plusieurs composés de formule (I) différents et d'un ou de plusieurs sels de composés de formule (I) différents,
- R¹ et R² signifiant indépendamment l'un de l'autre hydrogène, méthyle ou éthyle,
- R³ et R⁴ signifiant indépendamment l'un de l'autre hydrogène, hydroxy, méthoxy ou éthoxy, et
- la configuration de l'atome de carbone chiral étant (R) ou (S),
i) et ii) se présentant dans un mélange en un rapport l'un par rapport à l'autre tel que le goût sucré de la substance à goût sucré (A) ou de la substance à goût sucré et également amer (C) soit renforcé synergiquement.
